# FASCICULE DE BREVET EUROPEEN

(11) **EP 1 266 896 B1**
(45) Date de publication et mention de la délivrance du brevet: **17.01.2007**
(21) Numéro de dépôt: 02078578.8
(22) Date de dépôt: 26.12.2000
(51) Int. Cl.: C07D 401/12, A61P 9/00, C07D 401/06, C07D 405/14, A61K 31/445, C07D 491/06, C07D 403/12

(54) **Urées linéaires ou cycliques, leur procédé de préparation et les compositions pharmaceutiques qui les contiennent**
Lineare oder zyklische Harnstoffe, Verfahren zu ihrer Herstellung und sie enthaltende pharmazeutische Zusammensetzungen
Linear or cyclic ureas, process for their preparation and pharmaceutical compositions containing them

(30) Priorité: 30.12.1999 FR 9916701
(43) Date de publication de la demande: 18.12.2002
(62) Demande divisionnaire de: 00403673.7
(73) Titulaire: Les Laboratoires Servier, 92415 Courbevoie Cedex (FR)
(72) Inventeur: Peglion, Jean-Louis, 78110 Le Vesinet (FR); Poitevin, Christophe, 75019 Paris (FR); Vilaine, Jean-Paul, 92290 Chatenay-Malabry (FR); Villeneuve, Nicole, 92500 Rueil-Malmaison (FR); Bourguignon, Marie-Pierre, 78400 Chatou (FR); Thollon, Catherine, 75011 Paris (FR)

(56) Documents cités:
- EP-A- 0 597 112
- EP-A- 0 870 763
- WO-A-98/02435

## Description

La présente invention concerne de nouvelles urées linéaires ou cycliques, leur procédé de préparation et les compositions pharmaceutiques qui les contiennent.

Les composés de la présente invention sont utiles dans le traitement des maladies ou des conditions pathologiques dans lesquelles une dysfonction endothéliale est connue comme étant un mécanisme pathogène et/ou aggravant. Ces pathologies sont : l'athérosclérose, l'existence de facteurs de risque vasculaire (dyslipidémie, diabète, hypertension artérielle systémique), les différentes formes cliniques d'ischémie myocardique ou périphérique, l'insuffisance cardiaque et les différentes formes d'hypertension artérielle pulmonaire. Ces composés sont également utiles pour le traitement de patients qui subissent une transplantation cardiaque ou une reperméabilisation vasculaire telle qu'un pontage, une thrombolyse ou une dilatation artérielle avec ou sans stent.

Une diminution de la disponibilité vasculaire en monoxyde d'azote (NO) représente le mécanisme majeur de la dysfonction endothéliale observée dans les maladies et conditions pathologiques précédemment citées et explique son rôle pathogène (*Cardiovasc. Res.,* **1999,** 43, 572 ; *Coronary. Art. Dis.* **1999,** 10, 277 ; *Coronary. Art. Dis.,* **1999,** 10, 301 ; *Coronary. Art. Dis.,* **1999,** 10, 287 ; *Coronary. Art. Dis*., **1999**, 10, 295).

En effet, dans ces conditions pathologiques, la dysfonction endothéliale peut résulter de deux mécanismes principaux: 1) une insuffisance de production de NO liée à l'inhibition de la NO synthase endothéliale par des inhibiteurs endogènes tel l'ADMA (asymétrique diméthyle arginine) dont la concentration plasmatique augmente chez des patients présentant des facteurs de risque cardiovasculaire (*Cardiovasc. Res.,* **1999,** 43, 542 ; *Hypertension,* **1997,** 29, 242 ; *Circulation,* **1997,** 95, 2068), 2) une inactivation du NO par l'anion superoxyde (O₂⁻) dont la production est accrue dans des conditions pathologiques (*Cardiovasc. Res.,* **1999,** 43, 562 ; *Eur.J Biochem.* **1997**, 245, 541 ; *J. Clin. Invest.,* **1993**, 91 2546).

Le NO exerce dans les conditions normales des effets majeurs tels que: 1) la régulation de la vasomotricité artérielle par son effet vasodilatateur (*N Engl. J Med*., **1993,** 329, 2002 ; Nature, **1980,** 288*,* 373), 2) la limitation de l'adhésion et de l'agrégation plaquettaire (*Trends Pharmacol. Sci*., **1991**, 12, 87), 3) le contrôle de l'adhésion aux cellules endothéliales de leucocytes et de monocytes (*Proc. Natl Acad. Sci. USA,* **1991**, 88, 4651), 4) l'inhibition de la prolifération des cellules musculaires lisses vasculaires (*Cardiovasc. Res*., **1999**, 43, 580, *Circulation*, **1993**, 87 V51)
Ceci explique que la déficience en NO au niveau de la paroi artérielle favorise des phénomènes pathologiques comme la vasoconstriction, la thrombose, l'accumulation lipidique et la prolifération des cellules musculaires lisses vasculaires.

Des expériences *in vitro* ont permis de démontrer que les composés de la présente invention permettent de limiter la dysfonction endothéliale et la diminution de disponibilité vasculaire en NO qui ont été induites par des tests impliquant les deux mécanismes physiopathologiques déjà cités : l'inhibition de la NO synthase endothéliale et un stress oxydatif par production d'O₂⁻.

Ainsi, grâce à leur activité pharmacologique spécifique, capable de limiter le développement de la dysfonction endothéliale, les composés de la présente invention, outre le fait qu'ils soient nouveaux, sont utiles pour prévenir le développement, l'extension et les complications des lésions athéroscléreuses notamment chez les patients présentant un facteur de risque vasculaire (dyslipidémie, diabète, hypertension artérielle), pour traiter les différentes formes cliniques d'ischémie myocardique ou périphérique, l'insuffisance cardiaque, les différentes formes d'hypertension artérielle pulmonaire. Ces composés sont également utilisés pour prévenir les complications vasculaires (spasme, thrombose, resténose, athérosclérose accélérée) chez les patients qui subissent un pontage, une dilatation vasculaire avec ou sans stent ou d'autres formes de reperméabilisation vasculaire ainsi qu'une transplantation cardiaque.

Des composés de structure voisine ont été décrits dans la littérature. C'est le cas plus particulièrement de la demande de brevet WO 94/13659 qui revendique des composés présentant notamment une urée cyclique, lesdits composés étant utiles dans le traitement des maladies du système nerveux central telles que la dépression ou la psychose. De même, le brevet FR 2 338 940 décrit des dérivés présentant notamment un motif 1-{1-[2-hydroxy-3-(aryloxy)-propyl]-4-pipéridyl}-3-aryl-imidazolidine-2-one, et les revendique pour leur utilité dans le traitement de l'hypertension vasculaire. Le brevet EP 0 526 342 décrit de nouvelles (isoquinoléin-5-yl)sulfonamides, lesquelles sont utiles dans le traitement de l'ischémie myocardique. Enfin, la demande de brevet WO 98//02435 décrit des composés ayant notamment un motif 1-[(4-pipéridinyl)-alkyl aminocarbonyl]- tétra hydroquinoléine, utiles dans le traitement des maladies neurodégénératives et des troubles ischémiques.

Les composés de la présente invention se distinguent nettement de cet art antérieur, tant dans leur structure chimique même, que dans leur activité pharmacologique spécifique de la protection endothéliale.

Plus particulièrement, la présente invention concerne les composés de formule (I) : 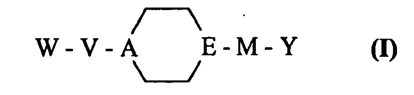 dans laquelle :
V représente une liaison simple ou une chaîne alkylène (C₁-C₆) linéaire ou ramifiée,
M représente une chaîne alkylène (C₁-C₄) linéaire ou ramifiée,
A et E représentent chacun un atome d'azote ou un groupement CH, mais au moins un des deux groupements A ou E représente un atome d'azote,
W représente un groupement de formule (ii) : 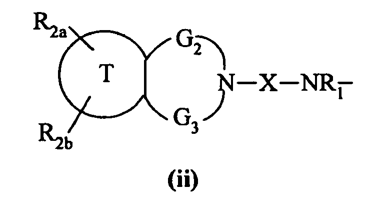
dans laquelle :
- X: représente un groupement carbonyle,
- G₂: représente une liaison simple ou un groupement méthylène,
- G₃: représente :
* une chaîne alkylène (C₂-C₃) linéaire quand G₂ représente une liaison,
* ou une chaîne alkylène (C₁-C₂) linéaire quand G₂ représente un groupement méthylène,
ladite chaîne alkylène dans chacun des cas comportant éventuellement une double liaison,
- T: représente un groupement phényle fusionné avec le cycle auquel il est lié,
- R₁: représente un atome d'hydrogène ou un groupement méthyle,
- R₂ₐ, R_{2b},: identiques ou différents, indépendamment l'un de l'autre, représentent un groupement choisi parmi atome d'hydrogène, atome d'halogène, groupement alkyle (C₁-C₆) linéaire ou ramifié, hydroxy, alkoxy (C₁-C₆) linéaire ou ramifié, mercapto, alkylthio (C₁-C₆) linéaire ou ramifié, trihalogénoalkyle (C₁-C₆) linéaire ou ramifié, cyano, nitro, amino, alkylamino (C₁-C₆) linéaire ou ramifié, dialkylamino (C₁-C₆) linéaire ou ramifié, trihalogénoalkoxy (C₁-C₆) linéaire ou ramifié, aryloxy, arylalkoxy (C₁-C₆) linéaire ou ramifié, alkylsulfonate (C₁-C₆) linéaire ou ramifié, trihalogénoalkylsulfonate (C₁-C₆) linéaire ou ramifié, alkylsulfonyle (C₁-C₆) linéaire ou ramifié,
ou R₂ₐ + R_{2b}, pris par deux en positions adjacentes, représente un groupement choisi parmi méthylènedioxy, 1,2-éthylènedioxy, 1,3-propylènedioxy, et éthylène éventuellement substitué par un groupement choisi parmi cyano, hydroxyméthyle, alkoxycarbonyle (C₁-C₆) linéaire ou ramifié, aryloxycarbonyle, et arylalkoxycarbonyle (C₁-C₆) linéaire ou ramifié,
- Y: représente un groupement aryloxy, hétéroaryloxy ou hétéroaryle-B, chacun de ces groupements étant éventuellement substitué par un ou plusieurs groupements, identiques ou différents, choisis parmi les définitions de R₂ₐ,
leurs hydrates, leurs solvates, ainsi que leurs sels d'addition à un acide pharmaceutiquement acceptable,
étant entendu que par groupement :
- aryle, on comprend un groupement choisi parmi phényle, biphényle, naphtyle, dihydronaphtyle, tétrahydronaphtyle, indanyle, indényle, et benzocyclobutyle,
- hétéroaryloxy, on comprend un système monocyclique aromatique ou bicyclique, lié à un atome d'oxygène, ledit système ayant de 5 à 12 chaînons, contenant un ou deux hétéroatomes, identiques ou différents, choisis parmi oxygène, azote et soufre, et dont l'un des cycles, dans le cas d'un système bicyclique possède un caractère aromatique, l'autre cycle pouvant être aromatique ou partiellement hydrogéné,
- hétéroaryle-B, on comprend un système monocyclique aromatique ou bicyclique aromatique, de 5 à 12 chaînons, contenant de 1 à 3 hétéroatomes, identiques ou différents, choisis parmi oxygène, azote et soufre,
- aryloxy, on comprend un groupement aryle tel que défini précédemment lié à un atome d'oxygène,
étant entendu que les composés de formule (I) sont différents de la 1-[2-[1-(1,4-benzodioxan-2-yl)méthyl-4-pipéridinyl] éthylaminocarbonyl]-1,2,3,4-tétrahydroquinoléine.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique.

Les substituants Y préférés, selon l'invention sont le groupement benzofuran-3-yl et le groupement phényloxy éventuellement substitué par un groupement R₂ₐ tel que défini dans la formule (I).

D'une façon particulièrement avantageuse, les composés préférés de l'invention sont les composés de formule (IB) : 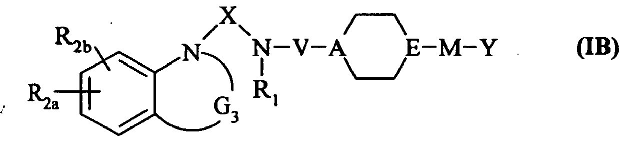 dans laquelle :
R₂ₐ et R_{2b} sont tels que définis dans la formule (I),
G₃ représente un groupement de formule -(CH₂)₂-, -(CH₂)₃- ou -CH=CH-,
X représente un groupement carbonyle,
R₁ représente un atome d'hydrogène,
A, E et V sont tels que définis dans la formule (I),
M représente une chaîne alkylène (C₁-C₄) linéaire ou ramifiée,
Y représente un groupement benzofuran-3-yl ou un groupement phényloxy éventuellement substitué par un groupement choisi parmi atome d'halogène, groupement alkylsulfonyle (C₁-C₆) linéaire ou ramifié, et alkylthio (C₁-C₆) linéaire ou ramifié.

D'une façon très préférentielle, les composés préférés de l'invention sont les composés de formule (IB) telle que définie précédemment dans laquelle :
* A représente un atome d'azote et E représente un groupement CH quand Y représente un groupement phényloxy éventuellement substitué,
* ou A représente un groupement CH et E représente un atome d'azote quand Y représente un groupement benzofuran-3-yl.

D'une autre façon préférentielle, les composés préférés de l'invention sont les composés de formule (IB) telle que définie précédemment dans laquelle V représente une liaison simple quand Y représente un groupement benzofuran-3-yl et V représente une chaîne alkylène (C₁-C₄) linéaire ou ramifiée quand Y représente un groupement phényloxy éventuellement substitué.

Les composés préférés de l'invention sont le :
- N- {2-[4-(phénoxyméthyl)-1-pipéridinyl]éthyl}-1-indolinecarboxamide,
- 5-fluoro-N-(2- {4-[2-(4-fluorophénoxy)éthyl]-1-pipéridinyl} éthyl)-1-indolecarboxamide,
- N- {2-[4-(*p*-fluorophénoxyméthyl)-1-pipéridinyl]éthyl}-1-indolinecarboxamide,
- et le N-{2-[4-(phénoxyméthyl)-1-pipéridinyl]éthyl}-1-indolecarboxamide.

Les hydrates, les solvates et les sels d'addition à un acide pharmaceutiquement acceptable des composés préférés font partie intégrante de l'invention.

L'invention s'étend également au procédé de préparation des composés de formule (I) caractérisé en ce qu'on utilise comme produit de départ un composé de formule (XV) : 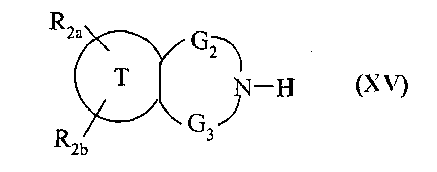 dans laquelle R₂ₐ, R_{2b}, G₂, G₃ et T ont les mêmes significations que dans la formule (I),
composé de formule (XV) que l'on traite soit par du diphosgène ou du triphosgène, en présence d'une base, soit par du di(1*H*-imidazol-1-yl)méthanone, pour conduire aux composés de formule (XVI) : 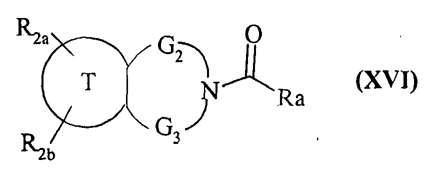 dans laquelle R₂ₐ, R_{2b}, G₂, G₃ et T sont tels que définis précédemment et Rₐ représente un atome de chlore, un groupement 1*H*-imidazol-1-yl ou un groupement de formule : 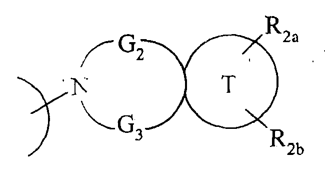 dans laquelle R₂ₐ, R_{2b}, G₂, G₃ et T sont tels que définis précédemment, composé de formule (XVI) qui est mis à réagir avec un composé de formule (XVII) : 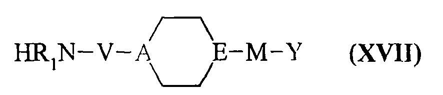 dans laquelle R₁, V, A, E, M et Y sont tels que définis dans la formule (I),
pour conduire aux composés de formule (I), que l'on purifie, le cas échéant, selon des techniques classiques de purification, dont on sépare éventuellement les isomères selon une technique classique de séparation, et que l'on transforme, si on le souhaite, en ses hydrates, ses solvates ou ses sels d'addition à un acide pharmaceutiquement acceptable.

Les composés de formule (XV) et (XVII) sont soit des composés commerciaux, soit obtenus selon des méthodes connues et classiques de la synthèse organique.

Les composés de la présente invention sont utiles dans le traitement des maladies ou des conditions pathologiques dans lesquelles une dysfonction endothéliale est connue. De ce fait, grâce à leur activité pharmacologique spécifique, les composés de l'invention sont utiles pour prévenir le développement, l'extension et les complications des lésions athéroscléreuses, en particulier chez les patients présentant un facteur de risque vasculaire (dyslipidémie, diabète, hypertension artérielle systémique), dans le traitement de l'ischémie myocardique ou périphérique, de l'insuffisance cardiaque, de l'hypertension artérielle pulmonaire, pour la prévention des complications vasculaires après pontage vasculaire, dilatation vasculaire, reperméabilisation vasculaire et transplantation cardiaque.

La présente invention a également pour objet les compositions pharmaceutiques contenant comme principe actif au moins un composé de formule (I), ses isomères optiques, ses hydrates, ses solvates, ses sels d'addition à un acide pharmaceutiquement acceptable, seul ou en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux, les gélules, les capsules, les suppositoires, les crèmes, les pommades, les gels dermiques, les préparations injectables ou buvables, les aérosols, les gouttes oculaires ou nasales, etc...

La posologie utile varie selon l'âge et le poids du patient, la voie d'administration, la nature et la sévérité de l'affection, et la prise de traitements éventuels associés et s'échelonne de 1 mg à 200 mg en une ou plusieurs prises par jour.

Les exemples suivants illustrent l'invention. Les produits de départ utilisés sont des produits connus ou préparés selon des modes opératoires connus. Les différentes préparations conduisent à des intermédiaires de synthèse utiles pour la préparation des composés de l'invention.

Les structures des composés décrits dans les exemples ont été déterminées selon les techniques spectrophotométriques usuelles (infrarouge, résonance magnétique nucléaire, spectrométrie de masse, ...).

Les points de fusion ont été déterminés soit à la platine chauffante de Kofler (K.), soit à la platine chauffante sous microscope (M.K.).

### PREPARATION 1 : 4-(p-fluorophénoxyméthyl)pipéridine

### Stade 1 : N-t-butyloxycarbonyl-4-(p-fluorophénoxyméthyl)pipéridine

Sur un mélange de 13,5 g de N-*t*-butyloxycarbonyl-4-hydroxyméthyl pipéridine, 7,45 g de *p*-fluorophénol, 17,4 g de triphénylphosphine et 140 ml de tétrahydrofurane sont versés, en 30 minutes, 10,5 ml d'azodicarboxylate de diéthyle. Après 12 heures d'agitation à température ambiante, le milieu réactionnel est concentré, repris à l'éther, lavé à l'eau puis à la soude 1*N*, puis lavé à l'eau. Après séchage, filtration et évaporation sous pression réduite, une chromatographie sur gel de silice (cyclohexane/acétate d'éthyle : 90/10) permet d'isoler le produit attendu.
***Point de fusion (K) : 94-98°C***

### Stade 2 : 4-(p-fluorophénoxyméthyl)pipéridine

12,1 g du produit obtenu au stade 1 est traité à température ambiante par 300 ml d'une solution d'éther chlorhydrique pendant 48 heures permettant d'obtenir le produit titre sous forme de chlorhydrate. La base libre est obtenue par traitement du chlorhydrate par de la soude 1*N* suivi d'une extraction au dichlorométhane.

### PREPARATION 2 : 4-[2-(p-fluorophénoxy)éthyl]pipéridine

Le produit est obtenu selon le procédé de la préparation 1 en utilisant au stade 1 le 2-(*N-t*-butyloxycarbonyl-4-pipéridinyl)éthanol comme substrat.

### PREPARATION 3 : 4-(phénoxyméthyl)pipéridine

Le produit est obtenu selon le procédé de la préparation 1 en utilisant au stade 1 le phénol à la place du *p*-fluorophénol.

### PREPARATION 4 : 4-(p-méthylsulfonylphénoxyméthyl)pipéridine

Le produit est obtenu selon le procédé de la préparation 1 en utilisant au stade 1 le *p*-méthylsulfonylphénol à la place du *p*-fluorophénol.
***Point de fusion (chlorhydrate) : 230-234°C***

### PREPARATION 6 : 2-{4-[(4-méthylsulfonylphénoxy)méthyl]-1-pipéridinyl} éthylamine

### Stade 1 : 1-t-butyloxycarbonyl-2-{4-[(4-méthylsulfonylphénoxy)méthyl]-1-pipéridinyl} éthylamine

Dans 50 ml de méthyl isobutylcétone sont additionnés 11,1 mmol du produit de la préparation 4, 11,1 mmol de 1-*t*-butyloxycarbonyl-2-bromo-éthylamine, 4,6 g de carbonate de sodium et une pointe de spatule d'iodure de potassium. Après 8 heures à reflux, puis 12 heures à température, un solide blanc est obtenu qui recristallise de l'acétonitrile permettant d'isoler le produit attendu.

### Stade 2 : 2-{4-[(4-méthylsulfonylphénoxy)méthyl]-1-pipéridinyl} éthylamine

On procède comme dans le stade 2 de la préparation 1.

### PREPARATION 7 : 1-[2-(benzofuran-3-yl)éthyl]-4-pipéridinylamine

### Stade 1 : N-t-butyloxycarbonyl-1-[2-(benzofuran-3-yl)éthyl]-4-pipéridinylamine

Dans 10 ml de toluène sont ajoutés 10 mmol de 2-(benzofuran-3-yl)-1-bromoéthane, 9,62 mmol de *t*-butyl-4-pipéridinylcarbamate, 2,65 g de carbonate de potassium et 0,65 g de sulfate de tétrabutylammonium. Après 12 heures à reflux, le milieu est repris à l'eau. La phase organique est ensuite lavée à l'eau, séchée puis filtrée. Une évaporation sous pression réduite permet d'isoler le produit attendu.

### Stade 2 : 1-[2-(benzofuran-3-yl)éthyl]-4-pipéridinylamine

On procède comme dans le stade 2 de la préparation 1.

### PREPARATION 8 : 1-[3-(benzofuran-3-yl)propyl]-4-pipéridinylamine

Le produit est obtenu selon le procédé de la préparation 7 en utilisant comme substrat au stade 1 le 3-(benzofuran-3-yl)-1-bromo-propane.

### PREPARATION 13 : 1-[2-(benzofuran-3-yl)éthyl]pipérazine

A 200 ml d'acétonitrile sont ajoutées 84 mmol de 2-(benzofuran-3-yl)-1-bromoéthane, 67 mmol de pipérazine et 56 mmol de carbonate de potassium. Après 4 heures à reflux, le milieu réactionnel est refroidi puis concentré. Le résidu est repris au dichlorométhane, lavé à l'eau, séché, filtré puis concentré sous pression réduite. Une chromatographie sur gel de silice (dichlorométhane/éthanol: 90/10) permet d'isoler le produit attendu.

### PREPARATION 15 : 1-[2-(p-flurophénoxy)éthyl]pipérazine

Le produit est obtenu selon le procédé de la préparation 13 en utilisant comme substrat le 1 -bromo-2-(*p*-fluorophénoxy)éthane.

### PREPARATION 17 : 2-{4-[2-(p-fluorophénoxy)éthyl]-1-pipéridinyl}éthylamine

### Stade 1 : {4-[2-(p-fluorophénoxy)éxhyl]-1-pipéridinyl}acétonitrile

Dans 320 ml de méthylisobutylcétone sont ajoutés 92,4 mmol du produit de la préparation 2, 6,5 ml de bromoacétonitrile, et 39,2 g de carbonate de potassium. Après 12 heures à reflux, le milieu réactionnel est filtré, concentré sous pression réduite. Le résidu est repris à l'eau et au dichlorométhane. La phase organique est lavée à l'eau, séchée, filtrée puis concentrée sous pression réduite permettant d'obtenir le produit attendu.
***Point de fusion (K) : 72°C***

### Stade 2: 2-{4-[2-(p-fluorophénoxy)éthyl]-1-pipéridinyl}éthylamine

A une suspension de 81 mmol de LiAlH₄ dans 200 ml de tétrahydrofurane, maintenue à 0°C, est additionnée une solution du produit obtenu au stade 1 dissout dans 200 ml de tétrahydrofurane. Après 12 heures d'agitation à température ambiante, le milieu réactionnel est hydrolysé par 2,75 ml d'eau, 2,2 ml de soude à 20 %, puis 10 ml d'eau. Après filtration et rinçage par du tétrahydrofurane, le filtrat est séché puis concentré sous pression réduite permettant d'isoler le produit attendu.

### PREPARATION 18 : 2-[4-(p-fluorophénoxyméthyl)-1-pipéridinyl]éthylamine

Le produit est obtenu selon le procédé de la préparation 17 en utilisant comme substrat le produit de la préparation 1.

### PREPARATION 19 : 2-[4-(phénoxyméthyl)-1-pipéridinyl]éthylamine

Le produit est obtenu selon le procédé de la préparation 17 en utilisant comme substrat le produit de la préparation 3.

### PREPARATION 20 : 2-{4-[2-(phénoxy)éthyl]-1-pipéridinyl}éthylamine

Le produit est obtenu selon le procédé de la préparation 17 en utilisant comme substrat la 4-(2-phénoxyéthyl)-pipéridine.

### PREPARATION 22 : 1-[2-(p-fluorophénoxy)éthyl]4-pipéridinylamine

Le produit est obtenu selon le procédé de la préparation 7 en utilisant comme substrat le 1-bromo-2-(*p*-fluorophénoxy)éthane.

### PREPARATION 41 : 2-[4-(p-Méthylthiophénoxyméthyl)-1-pipéridinyl] éthylamine

Le produit est obtenu selon le procédé de la préparation 17 en utilisant comme substrat la 4-(p-méthylthiophénoxyméthyl)pipéridine, ce produit étant obtenu selon le procédé de la préparation 1 en utilisant au stade 1 le p-(méthylthio)-phénol à la place du p-fluorophénol.
***Point de fusion (K.) : 60-66°C***

### PREPARATION 42 : N-Méthyl-2-[4-(phénoxyméthyl)-1-pipéridinyl]éthanamine

5 g de di-*tert*-butyl dicarbonate sont introduits dans une solution de 5 g du produit de la préparation 19 dans 40 ml de chlorure de méthylène et le mélange est agité pendant 24 heures. Après concentration sous vide, le milieu réactionnel est purifié par chromatographie flash (éluant : CH₂Cl₂/EtOH : 95/5). Le produit obtenu (5,5 g) est dissout dans 50 ml de THF et coulé sur une suspension de 1,7 g d'hydrure de lithium et d'aluminium dans 70 ml de THF. Le milieu réactionnel est porté à reflux pendant 7 heures puis après refroidissement, hydrolysé à 5°C successivement par 11,7 ml d'H₂O, 6,1 ml de soude à 20 % et encore 6,7 ml d'eau. Après filtration et concentration, on obtient le produit attendu.

### PREPARATION 43 : 2-{4-[2-(1-Benzofuran-3-yl)éthyl]-1-pipérazinyl}éthanamine

Le produit est obtenu sous forme d'une huile selon le procédé de la préparation 17 en utilisant comme substrat au stade 1 le produit de la préparation 13.

### PREPARATION 44 : 2-{4-[2-(4-Fluorophénoxy)éthyl]-1-pipérazinyl}éthanamine

Le produit est obtenu sous forme d'une huile incolore selon le procédé de la préparation 17 en utilisant comme substrat au stade 1 le produit de la préparation 15.

### EXEMPLE 24 : N-{1-[2-(p-Fluorophénoxy)éthyl]-4-pipéridinyl}indolinecarboxamide et son chlorhydrate

### Stade 1 : Chlorure d'indolin-1-yl carbonyle

22,5 mmol de diphosgène en solution dans 90 ml de dichlorométhane sont refroidies à 0°C, puis 45 mmol d'indoline sont ajoutées, puis 67,5 mmol de triéthylamine, tout en maintenant la température à 0°C. Après 12 heures à température ambiante, le milieu est filtré et le filrat est concentré sous pression réduite.

### Stade 2 : N-{1-[2-(p-Fluorophénoxy)éthyl]-4-pipéridinyl}indolinecarboxamide et son chlorhydrate

A une solution de 12 mmol du produit de la préparation 22, 36 mmol de diisopropyléthylamine dans 210 ml de dichlorométhane sont additionnées, par portions, 12 mmol du produit obtenu au stade 1. Après 48 heures à température ambiante, le milieu est dilué à l'eau, décanté, lavé à l'eau, séché, filtré et évaporé. Une chromatographie sur gel de silice (dichlorométhane/éthanol : 95/5) permet d'isoler le produit attendu qui est transformé en chlorhydrate dans l'éthanol chlorhydrique.
***Point de fusion : 228-235°C***

| ***Microanalyse élémentaire:*** | | | | |
|---|---|---|---|---|
| | ***C*** | ***H*** | ***N*** | ***Cl*** |
| ***% trouvé*** | ***61,70*** | ***6,36*** | ***9,69*** | ***9,62*** |
| ***% calculé*** | ***61,85*** | ***6,43*** | ***9,84*** | ***9,96*** |

### EXEMPLE 25 : N-{1-[3-(Benzofuran-3-yl)propyl]-4-pipéridinyl}-1-indolinecarboxamide et son chlorhydrate

Le produit est obtenu selon le procédé de l'exemple 24 en utilisant comme substrat au stade 2 le composé de la préparation 8 au lieu de celui de la préparation 22. Le produit obtenu est transformé en son chlorhydrate.
***Point de fusion (M.K.) : 253-257°C***

| ***Microanalyse élémentaire :*** | | | | |
|---|---|---|---|---|
| | ***C*** | ***H*** | ***N*** | ***Cl*** |
| ***% trouvé*** | ***68,48*** | ***6,78*** | ***9,56*** | ***8,08*** |
| ***% calculé*** | ***68,25*** | ***6,87*** | ***9,55*** | ***8,06*** |

### EXEMPLE 26 : N-{1-[2-(Benzofuran-3-yl)éthyl]-4-pipéridinyl}-1-indolinecarboxamide et son chlorhydrate

Le produit est obtenu selon le procédé de l'exemple 24 en utilisant comme substrat au stade 2 le composé de la préparation 7 au lieu de celui de la préparation 22. Le produit obtenu est transformé en son chlorhydrate par action d'une solution d'éther chlorhydrique.
***Point de fusion (M.K.) : 261-265°C***

| ***Microanalyse élémentaire :*** | | | | |
|---|---|---|---|---|
| | ***C*** | ***H*** | ***N*** | ***Cl*** |
| ***% trouvé*** | ***67,71*** | ***6,67*** | ***9,81*** | ***8,77*** |
| ***% calculé*** | ***67,67*** | ***6,63*** | ***9,86*** | ***8,32*** |

### EXEMPLE 27 : N-{1-[3-(Benzofuran-3-yl)propyl]-4-pipéridinyl}-1,2,3,4-tétrahydro-1-quinolinecarboxamide et son chlorhydrate

Le produit est obtenu selon le procédé de l'exemple 24 en utilisant comme substrat au stade 1 la 1,2,3,4-tétrahydroquinoline et au stade 2 le produit de la préparation 8 au lieu de celui de la préparation 22. Le produit obtenu est transformé en son chlorhydrate qui est recristallisé de l'éthanol.
***Point de fusion (M.K) : 227-231°C***

| ***Microanalyse élémentaire :*** | | | | |
|---|---|---|---|---|
| | ***C*** | ***H*** | ***N*** | ***Cl*** |
| ***% trouvé*** | ***69,13*** | ***7,13*** | ***9,26*** | ***7,79*** |
| ***% calculé*** | ***68,78*** | ***7,10*** | ***9,26*** | ***7,81*** |

### EXEMPLE 28 : N-{1-[2-(Benzofuran-3-yl)éthyl]-4-pipéridinyl}-1,2,3,4-tétrahydro-1-quinolinecarboxamide et son chlorhydrate

Le produit est obtenu selon le procédé de l'exemple 27 en utilisant comme substrat au stade 2 le composé de la préparation 7 au lieu de celui de la préparation 8. Le produit obtenu est transformé en son chlorhydrate qui recristallise de l'éthanol.
***Point de fusion (M.K.) : 215-219°C***

| ***Microanalyse élémentaire :*** | | | | |
|---|---|---|---|---|
| | ***C*** | ***H*** | ***N*** | ***Cl*** |
| ***% trouvé*** | ***68,27*** | ***6,85*** | ***9,44*** | ***8,26*** |
| ***% calculé*** | ***68,25*** | ***6,87*** | ***9,05*** | ***8,06*** |

### EXEMPLE 33 : N-(2-{4-[2-(p-Fluorophénoxy)éthyl]-1-pipéndinyl}éthyl)-1-indolinecarboxamide et son chlorhydrate

Le produit est obtenu selon le procédé de l'exemple 24 en utilisant comme substrat au stade 2 le composé de la préparation 17 au lieu de celui de la préparation 22. Le produit obtenu est transformé en son chlorhydrate.
***Point de fusion (M.K.) : 92-95°C***

| *Microanalyse élémentaire :* | | | | |
|---|---|---|---|---|
| | ***C*** | ***H*** | ***N*** | ***Cl*** |
| ***% trouvé*** | ***64,16*** | ***7,36*** | ***9,18*** | ***8,15*** |
| ***% calculé*** | ***64,35*** | ***6,97*** | ***9,38*** | ***7,91*** |

### EXEMPLE 34 : N-{2-[4-(p-Fluorophénoxyméthyl)-1-pipéridinyl]éthyl}-1-indolinecarboxamide et son chlorhydrate

Le produit est obtenu selon le procédé de l'exemple 24 en utilisant comme substrat au stade 2 le composé de la préparation 18 au lieu de celui de la préparation 22. Le produit obtenu est transformé en son chlorhydrate qui recristallise de l'acétonitrile.
***Point de fusion (M.K.) : 143-145°C***

| ***Microanalyse élémentaire :*** | | | | |
|---|---|---|---|---|
| | ***C*** | ***H*** | ***N*** | ***Cl*** |
| ***% trouvé*** | ***63,30*** | ***6,89*** | ***9,63*** | ***8,37*** |
| ***% calculé*** | ***63,66*** | ***6,74*** | ***9,68*** | ***8,17*** |

### EXEMPLE 35 : N-{2-[4-(Phénoxyméthyl)-1-pipéridinyl]éthyl}-1-indolinecarboxamide et son chlorhydrate

Le produit est obtenu selon le procédé de l'exemple 24 en utilisant comme substrat au stade 2 le composé de la préparation 19 au lieu de celui de la préparation 22. Le produit obtenu est transformé en son chlorhydrate qui recristallise de l'acétonitrile.
***Point de fusion (M.K.) : 205-210°C***

| ***Microanalyse élémentaire :*** | | | | |
|---|---|---|---|---|
| | ***C*** | ***H*** | ***N*** | ***Cl*** |
| ***% trouvé*** | ***65,93*** | ***7,29*** | ***9,97*** | ***8,87*** |
| ***% calculé*** | ***66,41*** | ***7,27*** | ***10,10*** | ***8,52*** |

### EXEMPLE 36: N-{2-[4-(p-Méthylsulfonylphénoxyméthyl)-1-pipéridinyl]éthyl}-1-indolinecarboxamide et son chlorhydrate

Le produit est obtenu selon le procédé de l'exemple 24 en utilisant comme substrat au stade 2 le composé de la préparation 6 au lieu de celui de la préparation 22. Le produit obtenu est transformé en son chlorhydrate qui recristallise de l'acétonitrile.
***Point de fusion (M.K.) : 199-203°C***

| ***Microanalyse élémentaire :*** | | | | | |
|---|---|---|---|---|---|
| | ***C*** | ***H*** | ***N*** | ***Cl*** | ***S*** |
| ***% trouvé*** | ***58,54*** | ***6,64*** | ***8,56*** | ***7,25*** | ***6,32*** |
| ***% calculé*** | ***58,35*** | ***6,53*** | ***8,51*** | ***7,18*** | ***6,49*** |

### EXEMPLE 37 : N-(2-{4-[(2-(Phénoxy)éthyl]-1-pipéridinyl}éthyl)-1-indolinecarboxamide et son chlorhydrate

Le produit est obtenu selon le procédé de l'exemple 24 en utilisant comme substrat au stade 2 le composé de la préparation 20 au lieu de celui de la préparation 22. Le produit obtenu est transformé en son chlorhydrate dans l'éthanol chlorhydrique.
***Point de fusion (M.K.) : 83-87°C***

| ***Microanalyse élémentaire :*** | | | | |
|---|---|---|---|---|
| | ***C*** | ***H*** | ***N*** | ***Cl*** |
| ***% trouvé*** | ***67,69*** | ***7,96*** | ***9,82*** | ***8,03*** |
| ***% calculé*** | ***67,04*** | ***7,50*** | ***9,77*** | ***8,25*** |

### EXEMPLE 38 : N-{1-[2-(Benzofuran-3-yl)éthyl]-4-pipéridinyl}-1-indolecarboxamide et son chlorhydrate

### Stade 1 : (Imidazol-1-yl-indol-1-yl)méthanone

A une solution de 85,4 mmol d'indole dans 305 ml d'acétonitrile sont ajoutés successivement 90 mmol de carbonyldiimidazole et 0,24 g de 4-diméthylaminopyridine. Après 8 heures à reflux, le milieu est concentré. Le résidu est repris par un minimum d'éther. Après filtration, on obtient le produit attendu.

### Stade 2 : N-{1-[2-(Benzofuran-3-yl)éthyl]-4-pipéridinyl}-1-indolecarboxamide et son chlorhydrate

Une solution du produit obtenu au stade 1, de produit de la préparation 7 dans 25 ml d'acétonitrile, est portée à reflux pendant 12 heures. Après refroidissement, le précipité formé est filtré. Le filtrat est concentré, repris par de l'acétate d'éthyle, lavé à l'eau. La phase organique est séchée, filtrée puis concentrée sous pression réduite. Une chromatographie sur gel de silice (dichlorométhane/éthanol : 95/5) permet d'isoler le produit attendu qui est transformé en chlorhydrate par une solution d'éthanol chlorhydrique.
***Point de fusion (M.K.) : 275-280°C***

| ***Microanalyse élémentaire :*** | | | | |
|---|---|---|---|---|
| | ***C*** | ***H*** | ***N*** | ***Cl*** |
| ***% trouvé*** | ***67,96*** | ***6,14*** | ***9,82*** | ***8,34*** |
| ***% calculé*** | ***68,00*** | ***6,18*** | ***9,91*** | ***8,36*** |

### EXEMPLE 39 : N-{2-[4-(p-Fluorophénoxyméthyl)-1-pipéridinyl]éthyl}-1-indolecarboxamide et son chlorhydrate

Le produit est obtenu selon le procédé de l'exemple 38 en utilisant comme substrat au stade 2 le composé de la préparation 18 au lieu de celui de la préparation 7. Le produit obtenu est transformé en son chlorhydrate par action d'une solution d'éther chlorhydrique.
***Point de fusion (M.K.) : 206-210°C***

| ***Microanalyse élémentaire :*** | | | | |
|---|---|---|---|---|
| | ***C*** | ***H*** | ***N*** | ***Cl*** |
| ***% trouvé*** | ***63,82*** | ***6,39*** | ***9,50*** | ***8,57*** |
| ***% calculé*** | ***63,96*** | ***6,30*** | ***9,73*** | ***8,21*** |

### EXEMPLE 40 : N-(2-{4-[2-(p-Fluorophénoxy)éthyl]-1-pipéridinyl}éthyl)-1-indolecarboxamide et son chlorhydrate

Le produit est obtenu selon le procédé de l'exemple 38 en utilisant comme substrat au stade 2 le composé de la préparation 17 au lieu de celui de la préparation 7. Le produit obtenu est transformé en son chlorhydrate par action d'une solution d'éthanol chlorhydrique.
***Point de fusion (M.K.) : 214-218°C***

| ***Microanalyse élémentaire :*** | | | | |
|---|---|---|---|---|
| | ***C*** | ***H*** | ***N*** | ***Cl*** |
| ***% trouvé*** | ***64,60*** | ***6,55*** | ***9,38*** | ***7,99*** |
| ***% calculé*** | ***64,64*** | ***6,55*** | ***9,42*** | ***7,95*** |

### EXEMPLE 41 : N-{2-[4-(Phénoxyméthyl)-1-pipéridinyl]éthyl}-1-indolecarboxamide et son chlorhydrate

Le produit est obtenu selon le procédé de l'exemple 38 en utilisant comme substrat au stade 2 le composé de la préparation 19 au lieu de celui de la préparation 7. Le produit obtenu est transformé en son chlorhydrate.
***Point de fusion (M.K.) : 171-174°C***

| ***Microanalyse élémentaire :*** | | | | |
|---|---|---|---|---|
| | ***C*** | ***H*** | ***N*** | ***Cl*** |
| ***% trouvé*** | ***66,41*** | ***6,88*** | ***10,01*** | ***8,85*** |
| ***% calculé*** | ***66,74*** | ***6,82*** | ***10,15*** | ***8,56*** |

### EXEMPLE 42 : N-{2-[4-(Phénoxyméthyl)-1-pipéridinyl]-éthyl}-5,6-diméthoxy-1-indolecarboxamide et son chlorhydrate

Le produit est obtenu selon le procédé de l'exemple 38 en utilisant au stade 1 la 5,6-diméthoxyindole au lieu de l'indole, et au stade 2 le produit de la préparation 19 au lieu de celui de la préparation 7. Le produit obtenu est transformé en son chlorhydrate qui cristallise de l'acétonitrile.
***Point de fusion (M.K.) : 204-208°C***

### EXEMPLE 43 : N-(2-{4-[2-(p-Fluorophénoxy)éthyl]-1-pipéridinyl}éthyl)-5-hydroxy-1-indolecarboxamide et son chlorhydrate

### Stade 1 : (5-benzyloxyindole-1-yl-imidazol-1-yl)-méthanone

Le produit est obtenu selon le procédé de l'exemple 38, stade 1, en utilisant le 5-benzyloxyindole à la place de l'indole.

### Stade 2 : N-(2-{4-[2-(p-Fluorophénoxy)éthyl]-1-pipéridinyl}éthyl)-5-benzyloxy-1-indolecarboxamide

Le produit est obtenu selon le procédé du stade 2 de l'exemple 38 en utilisant le produit de la préparation 17.

### Stade 3 : N-(2-{4-[2-(p-Fluorophénoxy)éthyl]-1-pipéridinyl}éthyl)-5-hydroxy-1-indolecarboxamide et son chlorhydrate

3,87 mmol du produit obtenu au stade 2 dans 150 ml de méthanol sont hydrogénées en présence de 0,4 g de Pd/C à 10 %. Après 4 heures à température ambiante et pression atmosphérique, le milieu est concentré sous pression réduite. Une chromatographie sur gel de silice (dichlorométhane/éthanol : 95/5) permet d'isoler le produit attendu. Le produit est transformé en son chlorhydrate par action d'une solution d'éther chlorhydrique, lequel cristallise de l'acétonitrile.
***Point de fusion (M.K.) : 237-241°C***

### EXEMPLE 44 : N-(2-{4-[2-(p-Fluorophénoxy)éthyl]-1-pipéridinyl}éthyl)-5-hydroxy-1-indolinecarboxamide et son chlorhydrate

Le produit est obtenu selon le procédé de l'exemple 43 en utilisant comme substrat au stade 1 la (5-benzyloxyindolin-1-yl)-imidazol-1-yl-méthanone. Le produit obtenu est transformé en son chlorhydrate par action d'une solution d'éther chlorhydrique.
***Point de fusion (M.K.) : 193-196°C***

### EXEMPLE 45 : N-{2-[4-(Phénoxyméthyl)-1-pipéridinyl]éthyl}-5-fluoro-1-indolecarboxamide et son chlorhydrate

### Stade 1 : bis-(5-Fluoroindole-1-yl)méthanone

A une solution de 74 mmol de 5-fluoroindole dans 300 ml d'acétonitrile sont additionnées 78,4 mmol de carbonyldiimidazole et 0,2 g de 4-diméthylaminopyridine. Après 8 heures à reflux, puis concentration, le résidu est repris dans un minimum d'éther. On filtre le produit qui a concrétisé et qui correspond au produit attendu.

### Stade 2 : N-{2-[4-(Phénoxyméthyl)-1-pipéridinyl]éthyl}-5-fluoro-1-indolecarboxamide et son chlorhydrate

Le produit est obtenu selon le procédé de l'exemple 38 en utilisant comme substrat le produit obtenu au stade 1 et le composé de la préparation 19 au lieu de celui de la préparation 17. Le produit obtenu est transformé en son chlorhydrate par action d'éther chlorhydrique et il cristallise de l'acétonitrile.
***Point de fusion (M.K.) : 228-234°C***

| ***Microanalyse élémentaire :*** | | | | |
|---|---|---|---|---|
| | ***C*** | ***H*** | ***N*** | ***Cl*** |
| ***% trouvé*** | ***64,04*** | ***6,32*** | ***9,71*** | ***7,88*** |
| ***% calculé*** | ***63,96*** | ***6,30*** | ***9,73*** | ***8,21*** |

### EXEMPLE 46 : N-(2-{4-[2-(p-Fluorophénoxy)éthyl]-1-pipéridinyl}éthyl)-5-fluoro-1-indolecarboxamide et son chlorhydrate

Le produit est obtenu selon le procédé de l'exemple 45 en utilisant comme substrat au stade 2 le composé de la préparation 17 au lieu de celui de la préparation 19. Le produit obtenu est transformé en son chlorhydrate par action de l'éther chlorhydrique et il cristallise de l'isopropanol.
***Point de fusion (M.K.) : 205-209°C***

| ***Microanalyse élémentaire :*** | | | | |
|---|---|---|---|---|
| | ***C*** | ***H*** | ***N*** | ***Cl*** |
| ***% trouvé*** | ***62,12*** | ***6,17*** | ***8,93*** | ***7,39*** |
| ***% calculé*** | ***62,13*** | ***6,08*** | ***9,06*** | ***7,64*** |

### EXEMPLE 69 : 6-Fluoro-N-(2-{4-[(4-fluorophénoxy)méthyl]-1-pipéridinyl}éthyl)-1H-indole-1-carboxamide et son chlorhydrate

Le produit est obtenu selon le procédé de l'exemple 45, des stades 1 à 2, en utilisant comme substrat au stade 1 le 6-fluoroindole à la place du 5-fluoroindole et au stade 2 le composé de la préparation 18. Le produit est transformé en son chlorhydrate par action d'une solution d'éther chlorhydrique.
***Point de fusion (M,K.) : 180-184°C***

| ***Microanalyse élémentaire :*** | | | | |
|---|---|---|---|---|
| | ***C*** | ***H*** | ***N*** | ***Cl*** |
| ***% trouvé*** | ***61,27*** | ***5,77*** | ***9,34*** | ***8,23*** |
| ***% calculé*** | ***61,40*** | ***5,82*** | ***9,34*** | ***7,88*** |

### EXEMPLE 70 : 6-Fluoro-N-(2-{4-[2-(4-fluorophénoxy)éthyl]-1-pipéridinyl} éthyl)-1H-indole-1-carboxamide et son chlorhydrate

Le produit est obtenu selon le procédé de l'exemple 45, des stades 1 à 2, en utilisant comme substrat au stade 1 le 6-fluoroindole à la place du 5-fluoroindole et au stade 2 le composé de la préparation 17. Le produit est transformé en son chlorhydrate par action d'une solution d'éther chlorhydrique.
***Point de fusion (M.K.) : 185-190°C***

| ***Microanalyse élémentaire :*** | | | | |
|---|---|---|---|---|
| | ***C*** | ***H*** | ***N*** | ***Cl*** |
| ***% trouvé*** | ***61,99*** | ***6,08*** | ***9,01*** | ***7,75*** |
| ***% calculé*** | ***62,13*** | ***6,08*** | ***9,06*** | ***7,64*** |

### EXEMPLE 71 : 6-Chloro-N-{2-[4-(phénoxyméthyl)-1-pipéridinyl]éthyl}-1H-indole-1-carboxamide et son chlorhydrate

Le produit est obtenu selon le procédé de l'exemple 45, des stades 1 à 2, en utilisant comme substrat au stade 1 le 6-chloroindole à la place du 5-fluoroindole et au stade 2 le composé de la préparation 19. Le produit est transformé en son chlorhydrate par action d'une solution d'éther chlorhydrique.
***Point de fusion (M.K.) : 211-215°C***

| ***Microanalyse élémentaire :*** | | | | |
|---|---|---|---|---|
| | ***C*** | ***H*** | ***N*** | ***Cl*** |
| ***% trouvé*** | ***61,72*** | ***6,13*** | ***9,39*** | ***15,91*** |
| ***% calculé*** | ***61,61*** | ***6,07*** | ***9,37*** | ***15,81*** |

### EXEMPLE 72 : 6-Chloro-N-(2-{4-[2-(4-fluorophénoxy)éthyl]-1-pipéridinyl} éthyl)-1H-indole-1-carboxamide et son chlorhydrate

Le produit est obtenu selon le procédé de l'exemple 45, des stades 1 à 2, en utilisant comme substrat au stade 1 le 6-chloroindole à la place du 5-fluoroindole et au stade 2 le composé de la préparation 17. Le produit est transformé en son chlorhydrate par action d'une solution d'éther chlorhydrique.
***Point de fusion (M.K.) : 176-180°C***

| ***Microanalyse élémentaire :*** | | | | |
|---|---|---|---|---|
| | ***C*** | ***H*** | ***N*** | ***Cl*** |
| ***% trouvé*** | ***60,36*** | ***5,99*** | ***8,61*** | ***14,98*** |
| ***% calculé*** | ***60,00*** | ***5,87*** | ***8,75*** | ***14,76*** |

### EXEMPLE 73 : 5-Chloro-N-{2-[4-(phéuoxyméthyl)-1-pipéridinyl]éthyl}-1H-indole-1-carboxamide et son chlorhydrate

Le produit est obtenu selon le procédé de l'exemple 45, des stades 1 à 2, en utilisant comme substrat au stade 1 le 5-chloroindole à la place du 5-fluoroindole et au stade 2 le composé de la préparation 19. Le produit est transformé en son chlorhydrate par action d'une solution d'éther chlorhydrique.
***Point de fusion (M.K.) : 187-190°C***

| ***Microanalyse élémentaire :*** | | | | |
|---|---|---|---|---|
| | ***C*** | ***H*** | ***N*** | ***Cl*** |
| ***% trouvé*** | ***61,82*** | ***6,12*** | ***9,35*** | ***15,54*** |
| ***% calculé*** | ***61,61*** | ***6,07*** | ***9,37*** | ***15,81*** |

### EXEMPLE 74: 6-Méthoxy-N-{2-[4-(phénoxyméthyl)-1-pipéridinyl]éthyl}-1H-indole-1-carboxamide et son chlorhydrate

Le produit est obtenu selon le procédé de l'exemple 38, des stades 1 à 2, en utilisant comme substrat au stade 1 le 6-méthoxyindole et au stade 2 le composé de la préparation 19. Le produit est transformé en son chlorhydrate par action d'une solution d'éther chlorhydrique.
***Point de fusion (M.K.) : 201-207°C***

| ***Microanalyse élémentaire :*** | | | | |
|---|---|---|---|---|
| | ***C*** | ***H*** | ***N*** | ***Cl*** |
| ***% trouvé*** | ***64,96*** | ***6,87*** | ***9,33*** | ***8,19*** |
| ***% calculé*** | ***64,93*** | ***6,81*** | ***9,46*** | ***7,99*** |

### EXEMPLE 75 : 5-Méthoxy-N-{2-[4-(phénoxyméthyl)-1-pipéridinyl]éthyl}-1H-indole-1-carboxamide et son chlorhydrate

Le produit est obtenu selon le procédé de l'exemple 38, des stades 1 à 2, en utilisant comme substrat au stade 1 le 5-méthoxyindole et au stade 2 le composé de la préparation 19. Le produit est transformé en son chlorhydrate par action d'une solution d'éther chlorhydrique.
***Point de fusion (M.K.) : 178-182°C***

| ***Microanalyse élémentaire :*** | | | | |
|---|---|---|---|---|
| | ***C*** | ***H*** | ***N*** | ***Cl*** |
| ***% trouvé*** | ***65,12*** | ***7,00*** | ***9,48*** | ***8,02*** |
| ***% calculé*** | ***64,93*** | ***6,81*** | ***9,46*** | ***7,99*** |

### EXEMPLE 76 : N-(2-{4-[(4-Fluorophénoxy)méthyl]-1-pipéridinyl}éthyl)-5-méthoxy-1H-indole-1-carboxamide et son chlorhydrate

Le produit est obtenu selon le procédé de l'exemple 38, des stades 1 à 2, en utilisant comme substrat au stade 1 le 5-méthoxyindole et au stade 2 le composé de la préparation 18. Le produit est transformé en son chlorhydrate par action d'une solution d'éther chlorhydrique.
***Point de fusion (M.K.) : 186-191°C***

| ***Microanalyse élémentaire :*** | | | | |
|---|---|---|---|---|
| | ***C*** | ***H*** | ***N*** | ***Cl*** |
| ***% trouvé*** | ***62,38*** | ***6,41*** | ***8,90*** | ***8,10*** |
| ***% calculé*** | ***62,40*** | ***6,33*** | ***9,10*** | ***7,67*** |

### EXEMPLE 77 : N-(2-{4-[2-(4-Fluorophénoxy)éthyl]-1-pipéridinyl}éthyl)-5-méthoxy-1H-indole-1-carboxamide et son chlorhydrate

Le produit est obtenu selon le procédé de l'exemple 38, des stades 1 à 2, en utilisant comme substrat au stade 1 le 5-méthoxyindole et au stade 2 le composé de la préparation 17. Le produit est transformé en son chlorhydrate par action d'une solution d'éther chlorhydrique.
***Point de fusion (M.K.) : 203-207°C***

| ***Microanalyse élémentaire :*** | | | | |
|---|---|---|---|---|
| | ***C*** | ***H*** | ***N*** | ***Cl*** |
| ***% trouvé*** | ***63,02*** | ***6,46*** | ***8,78*** | ***7,43*** |
| ***% calculé*** | ***63,08*** | ***6,56*** | ***8,83*** | ***7,45*** |

### EXEMPLE 78 : 5-Hydroxy-N-{2-[4-(phénoxyméthyl)-1-pipéridinyl]éthyl}-1H-indole-1-carboxamide et son chlorhydrate

Le produit est obtenu selon le procédé de l'exemple 43, des stades 1 à 3, en utilisant comme substrat au stade 2 le composé de la préparation 19. Le produit est transformé en son chlorhydrate par action d'une solution d'éther chlorhydrique.
***Point de fusion (M.K.) : 210-214°C***

| ***Microanalyse élémentaire :*** | | | | |
|---|---|---|---|---|
| | ***C*** | ***H*** | ***N*** | ***Cl*** |
| ***% trouvé*** | ***64,42*** | ***6,73*** | ***9,86*** | ***8,54*** |
| ***% calculé*** | ***64,25*** | ***6,56*** | ***9,77*** | ***8,25*** |

### EXEMPLE 79 : 5-Chloro-N-{2-[4-(phénoxyméthyl)-1-pipéridinyl]éthyl}-1-indolinecarboxamide et son chlorhydrate

Le produit est obtenu selon le procédé de l'exemple 24, des stades 1 à 2, en utilisant comme substrat au stade 1 la 5-chloroindoline et au stade 2 le composé de la préparation 19. Le produit est transformé en son chlorhydrate par action d'une solution d'éther chlorhydrique.
***Point de fusion (M.K.) : 128-132°C***

| ***Microanalyse élémentaire :*** | | | | |
|---|---|---|---|---|
| | ***C*** | ***H*** | ***N*** | ***Cl*** |
| ***% trouvé*** | ***61,16*** | ***6,53*** | ***9,25*** | ***7,44*** |
| ***% calculé*** | ***61,33*** | ***6,49*** | ***9,33*** | ***7,87*** |

### EXEMPLE 80: 6-Chloro-N-(2-{4-[2-(4-fluorophénoxy)éthyl]-1-pipéridinyl} éthyl)-1-indolinecarboxamide et son chlorhydrate

Le produit est obtenu selon le procédé de l'exemple 24, des stades 1 à 2, en utilisant comme substrat au stade 1 la 6-chloroindoline et au stade 2 le composé de la préparation 17. Le produit est transformé en son chlorhydrate par action d'une solution d'éther chlorhydrique.
***Point de fusion (M.K.) : 153-156°C***

| ***Microanalyse élémentaire :*** | | | | |
|---|---|---|---|---|
| | ***C*** | ***H*** | ***N*** | ***Cl*** |
| ***% trouvé*** | ***59,38*** | ***6,31*** | ***8,66*** | ***14,57*** |
| ***% calculé*** | ***59,75*** | ***6,27*** | ***8,71*** | ***14,70*** |

### EXEMPLE 81 : 5-Fluoro-N-(2-{4-[2-(4-fluorophénoxy)éthyl]-1-pipéridinyl} éthyl)-1-indolinecarboxamide et son chlorhydrate

Le produit est obtenu selon le procédé de l'exemple 24, des stades 1 à 2, en utilisant comme substrat au stade 1 la 5-fluoroindoline et au stade 2 le composé de la préparation 17. Le produit est transformé en son chlorhydrate par action d'une solution d'éther chlorhydrique.
***Point de fusion (M.K.) : 185-189°C***

| ***Microanalyse élémentaire :*** | | | | |
|---|---|---|---|---|
| | ***C*** | ***H*** | ***N*** | ***Cl*** |
| ***% trouvé*** | ***62,19*** | ***6,71*** | ***8,83*** | ***7,86*** |
| ***% calculé*** | ***61,86*** | ***6,49*** | ***9,02*** | ***7,61*** |

### EXEMPLE 82 : 6-Fluoro-N-{2-[4-(phénoxyméthyl)-1-pipéridinyl]éthyl}-1-indolinecarboxamide et son chlorhydrate

Le produit est obtenu selon le procédé de l'exemple 24, des stades 1 à 2, en utilisant comme substrat au stade 1 la 6-fluoroindoline et au stade 2 le composé de la préparation 19. Le produit est transformé en son chlorhydrate par action d'une solution d'éther chlorhydrique.
***Point de fusion (M.K.) : 168-172°C***

| ***Microanalyse élémentaire :*** | | | | |
|---|---|---|---|---|
| | ***C*** | ***H*** | ***N*** | ***Cl*** |
| ***% trouvé*** | ***63,34*** | ***6,73*** | ***9,55*** | ***8,80*** |
| ***% calculé*** | ***63,66*** | ***6,74*** | ***9,68*** | ***8,17*** |

### EXEMPLE 83 : 5-Fluoro-N-{2-[4-(phénoxyméthyl}-1-pipéridinyl]éthyl}-1-indolinecarboxamide et son chlorhydrate

Le produit est obtenu selon le procédé de l'exemple 24, des stades 1 à 2, en utilisant comme substrat au stade 1 la 5-fluoroindoline et au stade 2 le composé de la préparation 19. Le produit est transformé en son chlorhydrate par action d'une solution d'éther chlorhydrique.
***Point de fusion (M.K.) : 168-171°C***

| ***Microanalyse élémentaire :*** | | | | |
|---|---|---|---|---|
| | ***C*** | ***H*** | ***N*** | ***Cl*** |
| ***% trouvé*** | ***63,65*** | ***6,98*** | ***9,58*** | ***7,99*** |
| ***% calculé*** | ***63,66*** | ***6,74*** | ***9,68*** | ***8,17*** |

### EXEMPLE 84: 5-Méthoxy-N-{2-[4-(phénoxyméthyl)-1-pipéridinyl]éthyl}-1-indolinecarboxamide et son chlorhydrate

Le produit est obtenu selon le procédé de l'exemple 24, des stades 1 à 2, en utilisant comme substrat au stade 1 la 5-méthoxyindoline et au stade 2 le composé de la préparation 19. Le produit est transformé en son chlorhydrate par action d'une solution d'éther chlorhydrique.
***Point de fusion (M.K.) : 193-196°C***

| ***Microanalyse élémentaire :*** | | | | |
|---|---|---|---|---|
| | ***C*** | ***H*** | ***N*** | ***Cl*** |
| ***% trouvé*** | ***64,55*** | ***7,00*** | ***9,30*** | ***8,46*** |
| ***% calculé*** | ***64,64*** | ***7,29*** | ***9,42*** | ***7,95*** |

### EXEMPLE 85 : 5-Méthoxy-N-[2-(4-{[4-(méthylthio)phénoxy]méthyl}-1-pipéridinyl)éthyl]-1-indolinecarboxamide et son chlorhydrate

Le produit est obtenu selon le procédé de l'exemple 24, des stades 1 à 2, en utilisant comme substrat au stade 1 la 5-méthoxyindoline et au stade 2 le composé de la préparation 41. Le produit est transformé en son chlorhydrate par action d'une solution d'éther chlorhydrique.
***Point de fusion (M.K.) : 201-205°C***

| ***Microanalyse élémentaire** :* | | | | | |
|---|---|---|---|---|---|
| | ***C*** | ***H*** | ***N*** | ***Cl*** | ***S*** |
| ***% trouvé*** | ***60,96*** | ***7,15*** | ***8,45*** | ***7,59*** | ***6,53*** |
| ***% calculé*** | ***61,02*** | ***6,96*** | ***8,54*** | ***7,20*** | ***6,52*** |

### EXEMPLE 86 : N-[2-(4-{[4-(Méthylthio)phénoxy]méthyl}-1-pipéridinyl) éthyl]-1-indolinecarboxamide et son chlorhydrate

Le produit est obtenu selon le procédé de l'exemple 24, des stades 1 à 2, en utilisant comme substrat au stade 1 l'indoline et au stade 2 le composé de la préparation 41. Le produit est transformé en son chlorhydrate par action d'une solution d'éther chlorhydrique.
***Point de fusion (M.K.) : 165-170°C***

| ***Microanalyse élémentaire :*** | | | | | |
|---|---|---|---|---|---|
| | ***C*** | ***H*** | ***N*** | ***Cl*** | ***S*** |
| ***% trouvé*** | ***63,11*** | ***7,15*** | ***9,05*** | ***7,96*** | ***6,92*** |
| ***% calculé*** | ***62,39*** | ***6,98*** | ***9,09*** | ***7,67*** | ***6,94*** |

### EXEMPLE 87 : 5-Nitro-N-{2-[4-(phénoxyméthyl)-1-pipéridinyl]éthyl}-1-indolinecarboxamide et son chlorhydrate

Le produit est obtenu selon le procédé de l'exemple 24, des stades 1 à 2, en utilisant comme substrat au stade 1 la 5-nitroindoline et au stade 2 le composé de la préparation 19. Le produit est transformé en son chlorhydrate par action d'une solution d'éther chlorhydrique.
***Point de fusion (M.K.) : 210-220°C***

| ***Microanalyse élémentaire :*** | | | | |
|---|---|---|---|---|
| | ***C*** | ***H*** | ***N*** | ***Cl*** |
| ***% trouvé*** | ***59,86*** | ***6,27*** | ***12,01*** | ***8,10*** |
| ***% calculé*** | ***59,93*** | ***6,34*** | ***12,15*** | ***7,69*** |

### EXEMPLE 88 : N-(2-{4-[2-(4-Fluorophénoxy)éthyl]-1-pipéridinyl}éthyl)-5-nitro-1-indolinecarboxamide et son chlorhydrate

Le produit est obtenu selon le procédé de l'exemple 24, des stades 1 à 2, en utilisant comme substrat au stade 1 la 5-nitroindoline et au stade 2 le composé de la préparation 17. Le produit est transformé en son chlorhydrate par action d'une solution d'éther chlorhydrique.
***Point de fusion (M.K.) : 205-210°C***

| ***Microanalyse élémentaire :*** | | | | |
|---|---|---|---|---|
| | ***C*** | ***H*** | ***N*** | ***Cl*** |
| ***% trouvé*** | ***58,22*** | ***6,15*** | ***11,25*** | ***7,85*** |
| ***% calculé*** | ***58,47*** | ***6,13*** | ***11,36*** | ***7,19*** |

### EXEMPLE 89: 5,6-Diméthoxy-N-{2-[4-(phénoxyméthyl)-1-pipéridinyl]éthyl}-1-indolinecarboxamide et son chlorhydrate

Le produit de l'exemple 42 est soumis à une hydrogénation selon les conditions décrites dans le stade 3 de l'exemple 43 permettant d'isoler le produit attendu qui est transformé en son chlorhydrate de façon traditionnelle.
***Point de fusion (M.K.) : 118-122°C***

| ***Microanalyse élémentaire :*** | | | | |
|---|---|---|---|---|
| | ***C*** | ***H*** | ***N*** | ***Cl*** |
| ***% trouvé*** | ***63,50*** | ***7,39*** | ***8,79*** | ***7,71*** |
| ***% calculé*** | ***63,08*** | ***7,20*** | ***8,83*** | ***7,45*** |

### EXEMPLE 90 : N-{2-[4-(Phénoxyméthyl)-1-pipéridinyl]éthyl}-6,7-dihydro-5H-[1,3]dioxolo[4,5-f]indole-5-carboxamide et son chlorhydrate

Le produit est obtenu selon le procédé de l'exemple 24, des stades 1 à 2, en utilisant comme substrat au stade 1 la 5,6-méthylènedioxyindoline et au stade 2 le composé de la préparation 19. Le produit est transformé en son chlorhydrate par action d'une solution d'éther chlorhydrique.
***Point de fusion (M.K.) : 215-219°C***

| ***Microanalyse élémentaire :*** | | | | |
|---|---|---|---|---|
| | ***C*** | ***H*** | ***N*** | ***Cl*** |
| ***% trouvé*** | ***62,80*** | ***6,56*** | ***9,05*** | ***8,03*** |
| ***% calculé*** | ***62,67*** | ***6,57*** | ***9,14*** | ***7,71*** |

### EXEMPLE 91 : N-(2-{4-[2-(4-Fluorophénoxy)éthyl]-1-pipéridinyl}éthyl)-1,3-dihydro-2H-isoindole-2-carboxamide et son chlorhydrate

Le produit est obtenu selon le procédé de l'exemple 24, des stades 1 à 2, en utilisant comme substrat au stade 1 l'isoindoline et au stade 2 le composé de la préparation 17. Le produit est transformé en son chlorhydrate par action d'une solution d'éther chlorhydrique.
***Point de fusion (M.K.) : 175-179°C***

| ***Microanalyse élémentaire :*** | | | | |
|---|---|---|---|---|
| | ***C*** | ***H*** | ***N*** | ***Cl*** |
| ***% trouvé*** | ***63,78*** | ***6,93*** | ***9,22*** | ***7,85*** |
| ***% calculé*** | ***64,35*** | ***6,97*** | ***9,38*** | ***7,91*** |

### EXEMPLE 92 : N-{2-[4-(Phénoxyméthyl)-1-pipéridinyl]éthyl}-1,3-dihydro-2H-isoindole-2-carboxamide et son chlorhydrate

Le produit est obtenu selon le procédé de l'exemple 24, des stades 1 à 2, en utilisant comme substrat au stade 1 l'isoindoline et au stade 2 le composé de la préparation 19. Le produit est transformé en son chlorhydrate par action d'une solution d'éther chlorhydrique.
***Point de fusion (M.K.) : 178-181°C***

| ***Microanalyse élémentaire :*** | | | | |
|---|---|---|---|---|
| | ***C*** | ***H*** | ***N*** | ***Cl*** |
| ***% trouvé*** | ***66,03*** | ***7,26*** | ***10,00*** | ***8,57*** |
| ***% calculé*** | ***66,41*** | ***7,27*** | ***10,10*** | ***8,52*** |

### EXEMPLE 93 : N-{2-[4-(Phénoxyméthyl)-1-pipéndinyl]éthyl}-1,2,3,4-tétrahydro-2-isoquinolinecarboxamide et son hémi-fumarate

Le produit est obtenu selon le procédé de l'exemple 24, des stades 1 à 2, en utilisant comme substrat au stade 1 la 1,2,3,4-tétrahydroisoquinoline et au stade 2 le composé de la préparation 19. Le produit est transformé en son hémi-fumarate.
***Point de fusion (M.K.) : 150-154°C***

| ***Microanalyse élémentaire :*** | | | |
|---|---|---|---|
| | ***C*** | ***H*** | ***N*** |
| ***% trouvé*** | ***68,34*** | ***7,35*** | ***9,01*** |
| ***% calculé*** | ***68,45*** | ***7,28*** | ***9,07*** |

### EXEMPLE 94 : 6,7-Diméthoxy-N-{2-[4-(phénoxyméthyl)-1-pipéridinyl]éthyl}-1,2,3,4-tétrahydro-2-isoquinolinecarboxamide et son fumarate

Le produit est obtenu selon le procédé de l'exemple 24, des stades 1 à 2, en utilisant comme substrat au stade 1 la 6,7-diméthoxy-1,2,3,4-tétrahydroisoquinoline et au stade 2 le composé de la préparation 19. Le produit est transformé en son fumarate.
***Point de fusion (M.K.) : 200-205°C***

| ***Microanalyse élémentaire :*** | | | |
|---|---|---|---|
| | ***C*** | ***H*** | ***N*** |
| ***% trouvé*** | ***62,70*** | ***6,71*** | ***7,22*** |
| ***% calculé*** | ***63,25*** | ***6,90*** | ***7,38*** |

### EXEMPLE 95: 5-Hydroxy-N-{2-[4-(phénoxyméthyl)-1-pipéndinyl]éthyl}-1-indolinecarboxamide et son chlorhydrate

Le produit est obtenu selon le procédé de l'exemple 43, des stades 1 à 3, en utilisant comme substrat au stade 2 le composé de la préparation 19. Le produit est transformé en son chlorhydrate par action d'une solution d'éther chlorhydrique.
***Point de fusion (M.K.) : 198-202°C***

| ***Microanalyse élémentaire :*** | | | | |
|---|---|---|---|---|
| | ***C*** | ***H*** | ***N*** | ***Cl*** |
| ***% trouvé*** | ***63,80*** | ***7,33*** | ***9,64*** | ***8,38*** |
| ***% calculé*** | ***63,95*** | ***7,00*** | ***9,73*** | ***8,21*** |

### EXEMPLE 96 : 5-Hydroxy-N-méthyl-N-{2-[4-(phénoxyméthyl)-1-pipéridinyl] éthyl}-1-indolinecarboxamide et son chlorhydrate

Le produit est obtenu selon le procédé de l'exemple 43, des stades 1 à 3, en utilisant comme substrat au stade 2 le composé de la préparation 42. Le produit est transformé en son chlorhydrate par action d'une solution d'éther chlorhydrique.
***Point de fusion (M.K.) : 105-110°C***

| ***Microanalyse élémentaire :*** | | | | |
|---|---|---|---|---|
| | ***C*** | ***H*** | ***N*** | ***Cl*** |
| ***% trouvé*** | ***64,74*** | ***7,34*** | ***9,32*** | ***8,19*** |
| ***% calculé*** | ***64,63*** | ***7,23*** | ***9,20*** | ***7,95*** |

### EXEMPLE 97 : N-(2-{4-[2-(1-Benzofuran-3-yl)éthyl]-1-piperazinyl}éthyl)-1-indoline-2-carboxamide et son chlorhydrate

Le produit est obtenu selon le procédé de l'exemple 24, des stades 1 à 2, en utilisant comme substrat au stade 1 l'indoline et au stade 2 le composé de la préparation 43. Le produit est transformé en son chlorhydrate par action d'une solution d'éther chlorhydrique.
***Point de fusion (M.K.) : 195-199°C***

| ***Microanalyse élémentaire :*** | | | | |
|---|---|---|---|---|
| | ***C*** | ***H*** | ***N*** | ***Cl*** |
| ***% trouvé*** | ***61,40*** | ***6,43*** | ***11,26*** | ***14,66*** |
| ***% calculé*** | ***61,10*** | ***6,56*** | ***11,40*** | ***14,43*** |

### EXEMPLE 101 : 6-Chloro-N-(2-{4-[2-(phénoxy)méthyl]-1-pipéridinyl}éthyl)-1-indoline carboxamide et son chlorhydrate

Le produit est obtenu selon le procédé de l'exemple 24, des stades 1 à 2, en utilisant comme substrat au stade 1 la 6-chloroindoline et au stade 2 le composé de la préparation 19. Le produit est transformé en son chlorhydrate par action d'une solution d'éther chlorhydrique.
***Point de fusion (M.K.) : 175-179°C***

| ***Microanalyse élémentaire :*** | | | | |
|---|---|---|---|---|
| | ***C*** | ***H*** | ***N*** | ***Cl*** |
| ***% trouvé*** | ***61,32*** | ***6,58*** | ***9,32*** | ***16,53*** |
| ***% calculé*** | ***61,33*** | ***6,49*** | ***9,33*** | ***15,74*** |

### EXEMPLE 102 : 5-Fluoro-N-(2-{4-[2-(4-fluorophénoxy)éthyl]-1-piperazinyl} éthyl)-1-indoline carboxamide et son dichlorhydrate

Le produit est obtenu selon le procédé de l'exemple 24, des stades 1 à 2, en utilisant comme substrat au stade 1 la 5-fluororoindoline et au stade 2 le composé de la préparation 44. Le produit est transformé en son dichlorhydrate.
***Point de fusion (M.K.) : 179-184°C***

| ***Microanalyse élémentaire :*** | | | | |
|---|---|---|---|---|
| | ***C*** | ***H*** | ***N*** | ***Cl*** |
| ***% trouvé*** | ***54,67*** | ***5,89*** | ***11,18*** | ***14,16*** |
| ***% calculé*** | ***54,88*** | ***6,01*** | ***11,13*** | ***14,08*** |

### EXEMPLE 103 : N-{2-[4-(Phénoxyméthyl)-1-pipéridinyl]éthyl}-6-fluoro-1-indolecarboxamide et son chlorhydrate

Le produit est obtenu selon le procédé de l'exemple 38, des stades 1 à 2, mais en utilisant au stade 1 le 6-fluoroindole à la place de l'indole et au stade 2, le produit de la préparation 19.
***Point de fusion (M.K.) : 184-188°C***

### EXEMPLE 104 : N-Méthyl-N-{2-[4-(phénoxyméthyl)-1-pipéridinyl]éthyl}-5,6-diméthoxy 1-indolinecarboxamide et son chlorhydrate

Ce produit est obtenu selon le procédé de l'exemple 24, des stades 1 à 2, en utilisant comme substrat au stade 1 la 5,6-diméthoxyindoline et au stade 2 le composé de la préparation 42.
***Point de fusion (M.K) : 84-87°C***

### EXEMPLE 105 : N-{2-[4-(Phénoxyméthyl)-1-pipéridinyl]éthyl}-4,5,6-triméthoxy-1-indolecarboxamide et son chlorhydrate.

Le produit est obtenu selon le procédé de l'exemple 38, des stades 1 à 2, mais en utilisant au stade 1 le 4,5,6-triméthoxyindole à la place de l'indole et au stade 2 le produit de la préparation 19.
***Point de fusion (M.K.) : 217-220°C***

### EXEMPLE 106 : N-{2-[4-(Phénoxyméthyl)-1-pipéridinyl]éthyl}-4,5,6-triméthoxy-1-indolinecarboxamide et son chlorhydrate

Le produit est obtenu selon le procédé de l'exemple 24, des stades 1 à 2, mais en utilisant au stade 1 la 4,5,6-triméthoxyindoline et au stade 2 le produit de la préparation 19.
***Point de fusion (M.K.) : 147-151°C***

### ETUDE PHARMACOLOGIQUE DES COMPOSES DE L'INVENTION

Dans des conditions standard *in vitro,* la relaxation à l'acétylcholine (ACh) d'anneaux aortiques, totalement dépendante de la présence de l'endothélium, est le reflet de la production de NO (stimulée par l'ACh) qui en diffusant au niveau des cellules musculaires lisses entraîne la relaxation artérielle *(Nature,* **1980,** 288, 373).
Les composés de l'invention ont été testés sur deux modèles mettant en jeu deux mécanismes différents impliqués dans la dysfonction endothéliale observée en pathologie :
- le premier modèle consiste à induire une inhibition de la relaxation à l'ACh par blocage de l'activité enzymatique (NOS endothéliale) responsable de la production de NO.
- le second modèle consiste à induire un stress oxydant *in vitro* par un système enzymatique générateur d'O₂⁻ (xanthine oxydase -XO et hypoxanthine -Hypo).

### EXEMPLE 111 : Effets protecteurs vasculaires vis-à-vis d'une dysfonction endothéliale induite par un inhibiteur de NOS

L'aorte thoracique de rat Wistar (325-375 g), anesthésié par voie intrapérinétonéale au pentobarbital sodique (30 mg/kg), est prélevée et disséquée en anneaux de 3 mm de longueur. Chaque anneau est suspendu à un capteur de tension isométrique connecté à un système d'acquisition et la tension initiale appliquée est de 2,5 g. La solution physiologique utilisée, thermostatée à 37°C et oxygénée (95 % O₂ + 5 % CO₂) est composée de (en mM) : NaCl 112,0 ; KCl 5,0 ; CaCl₂ 2,5 ; KH₂PO₄ 1,0 ; MgSO₄ 1,2 ; NaHCO₃ 25,0 ; glucose 11,5, Ca-EDTA 0,016.
Après une période de stabilisation de 90 minutes, les préparations sont contractées avec de la phényléphrine (PHE 10⁻⁶M) et relaxées par addition de 10⁻⁵M d'acétylcholine afin de vérifier l'intégrité de la couche endothéliale. Si celle-ci est confirmée, les préparations sont rincées et une concentration de produit à tester est additionnée au milieu suivie de 3.10⁻⁷M de N^{G}-niro-L-arginine (LNA). Les préparations sont à nouveau contractées avec de la phényléphrine et 30 minutes après les relaxations à l'acétylcholine (ACh-10⁻⁸M à 10⁻⁵M) sont évaluées en présence d'indométhacine (10⁻⁵M).
Les valeurs de relaxation sont exprimées en pourcentage par rapport à la contraction maximale à la PHE. Les effets protecteurs des composés vis-à-vis de la dysfonction endothéliale correspondent à la différence entre les pourcentages de relaxation maximale observée en présence ou en absence de produit.
A titre d'exemple, le composé de l'exemple 35 à 10⁻⁸, 3x10⁻⁸, 10⁻⁷M inhibe respectivement de 8, 26 et 29 % la dysfonction endothéliale induite par le LNA.

### EXEMPLE 112 : Effets protecteurs vasculaires vis-à-vis d'une dysfonction endothéliale induite par un système générateur de O₂⁻

Ce protocole, réalisé sur anneaux aortiques de lapins Néo-Zélandais (2,5-3 kg) est comparable au précédent hormis pour les points suivants : la tension initiale appliquée est de 5 g et l'association XO (3 mU/ml -Hypo (10⁻⁴M) est utilisée à la place du LNA.
A titre d'exemple, le composé de l'exemple 35 à 3x10⁻⁸M inhibe de 17 % la dysfonction endothéliale induite par l'association XO-Hypo.

### EXEMPLE 113 : Implication de la voie du NO dans les effets protecteurs vasculaires détectés : évaluation de la production aortique de GMPc

En diffusant au niveau des cellules musculaires lisses, le NO produit par les cellules endothéliales active la guanylate cyclase soluble ce qui entraîne une augmentation du GMP cyclique responsable de la relaxation.
Ce médiateur a donc été dosé sur les anneaux aortiques de rat afin de démontrer que les effets protecteurs des composés vis-à-vis de la dysfonction endothéliale sont médiés par une augmentation de la disponibilité en NO.
Les anneaux aortiques de rat sont préparés comme précédemment. Les effets d'une incubation de 30 minutes des composés de l'invention à différentes concentrations sont évalués sur la production de GMPc stimulée par l'ACh (10⁻⁵M - 1 minute) en présence de LNA (3x10⁻⁶M). Ces expériences sont réalisées en présence d'isobutyl méthyl xanthine (10⁻⁵M) afin d'éviter la dégradation du GMPc par les phosphodiestérases. Les anneaux sont congelés dans de l'azote liquide et maintenus à -80°C jusqu'au dosage. Le contenu en GMPc est évalué par radio-immunodosage et exprimé par rapport à la quantité de protéines contenues dans le tissu (dosage par la méthode de Bradford).
A titre d'exemple, le composé de l'exemple 35 à 10⁻⁷M augmente la production de GMPc stimulée par l'ACh en présence de LNA de 28,1 %.

### EXEMPLE 114 : Composition pharmaceutique - Comprimé

| Formule de préparation pour 1000 comprimés dosés à 10 mg | |
|---|---|
| Composé de l'exemple 35 | 10 g |
| Hydroxypropylcellulose | 2 g |
| Polyvinylpyrrolidone | 2 g |
| Amidon de blé | 10 g |
| Lactose | 100 g |
| Stéarate de magnésium | 3 g |

## Revendications

1. Composés de formule (I) : 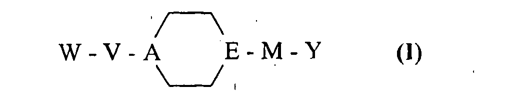 dans laquelle:
V représente une liaison simple ou une chaîne alkylène (C₁-C₆) linéaire ou ramifiée,
M représente une chaîne alkylène (C₁-C₄) linéaire ou ramifiée,
A et E représentent chacun un atome d'azote ou un groupement CH, mais au moins un des deux groupements A ou E représente un atome d'azote,
W représente un groupement de formule (ii) : 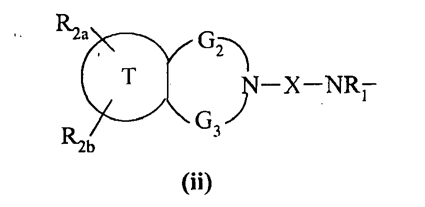
dans laquelle :
X représente un groupement carbonyle,
G₂ représente une liaison simple ou un groupement méthylène,
G₃ représente :
*une chaîne alkylène (C₂-C₃) linéaire quand G₂ représente une liaison,
*ou une chaîne alkylène (C₁-C₂) linéaire quand G₂ représente un groupement méthylène,
ladite chaîne alkylène dans chacun des cas comportant éventuellement une double liaison,
T représente un groupement phényle fusionné avec le cycle auquel il est lié,
R₁ représente un atome d'hydrogène ou un groupement méthyle,
R₂ₐ, R_{2b} , identiques ou différents, indépendamment l'un de l'autre, représentent un groupement choisi parmi atome d'hydrogène, atome d'halogène, groupement alkyle (C₁-C₆) linéaire ou ramifié, hydroxy, alkoxy (C₁-C₆) linéaire ou ramifié, mercapto, alkylthio (C₁-C₆) linéaire ou ramifié, trihalogénoalkyle (C₁-C₆) linéaire ou ramifié, cyano, nitro, amino, alkylamino (C₁-C₆) linéaire ou ramifié, dialkylamino (C₁-C₆) linéaire ou ramifié, trihalogénoalkoxy (C₁-C₆) linéaire ou ramifié, aryloxy, arylalkoxy (C₁-C₆) linéaire ou ramifié, alkylsulfonate (C₁-C₆) linéaire ou ramifié, trihalogénoalkylsulfonate (C₁-C₆) linéaire ou ramifié, alkylsulfonyle (C₁-C₆) linéaire ou ramifié,
ou R₂ₐ + R_{2b}, pris par deux en positions adjacentes, représente un groupement choisi parmi méthylènedioxy, 1,2-éthylènedioxy, 1,3-propylènedioxy, et éthylène éventuellement substitué par un groupement choisi parmi cyano, hydroxyméthyle, alkoxycarbonyle (C₁-C₆) linéaire ou ramifié, aryloxycarbonyle, et arylalkoxycarbonyle (C₁-C₆) linéaire ou ramifié,
Y représente un groupement aryloxy, hétéroaryloxy ou hétéroaryle-B, chacun de ces groupements étant éventuellement substitué par un ou plusieurs groupements, identiques ou différents, choisis parmi les définitions de R₂ₐ,
leurs hydrates, leurs solvates, ainsi que leurs sels d'addition à un acide pharmaceutiquement acceptable, étant entendu que par groupement :
- aryle, on comprend un groupement choisi parmi phényle, biphényle, naphtyle, dihydronaphtyle, tétrahydronaphtyle, indanyle, indényle, et benzocyclobutyle,
- hétéroaryloxy, on comprend un système monocyclique aromatique ou bicyclique lié à un atome d'oxygène, ledit système ayant de 5 à 12 chaînons, contenant un ou deux hétéroatomes, identiques ou différents, choisis parmi oxygène, azote et soufre, et dont l'un des cycles, dans le cas d'un système bicyclique possède un caractère aromatique, l'autre cycle pouvant être aromatique ou partiellement hydrogéné,
- hétéroaryle-B, on comprend un système monocyclique aromatique ou bicyclique aromatique, de 5 à 12 chaînons, contenant de 1 à 3 hétéroatomes, identiques ou différents, choisis parmi oxygène, azote et soufre,
- aryloxy, on comprend un groupement aryle tel que défini précédemment lié à un atome d'oxygène,
étant entendu que les composés de formule (I) sont différents de la 1-[2-[1-(1,4-benzodioxan-2-yl)méthyl-4-pipéridinyl]éthylaminocarbonyl]-1,2,3,4-tétrahydroquinoléine.

2. Composés de formule (I) selon la revendication 1 **caractérisés en ce que** Y représente un groupement benzofuran-3-yl ou un groupement phényloxy éventuellement substitué par un groupement R₂ₐ tel que défini dans la formule (I), leurs hydrates, leurs solvates et leurs sels d'addition à un acide pharmaceutiquement acceptable.

3. Composés de formule (I) selon la revendication 2 **caractérisés en ce qu**'ils représentent des composés de formule (IB) : 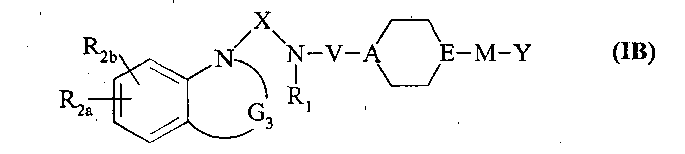 dans laquelle :
R₂ₐ et R_{2b} sont tels que définis dans la formule (I),
G₃ représente un groupement de formule -(CH₂)₂-, -(CH₂)₃- ou -CH=CH-,
X représente un groupement carbonyle,
R₁ représente un atome d'hydrogène,
A, E et V sont tels que définis dans la formule (I),
M représente une chaîne alkylène (C₁-C₄) linéaire ou ramifiée,
Y représente un groupement benzofuran-3-yl ou un groupement phényloxy éventuellement substitué par un groupement choisi parmi atome d'halogène, groupement alkylsulfonyle (C₁-C₆) linéaire ou ramifié, et alkylthio (C₁-C₆) linéaire ou ramifié,
leurs hydrates, leurs solvates et leurs sels d'addition à un acide pharmaceutiquement acceptable.

4. Composés de formule (1B) selon la revendication 3 **caractérisés en ce que** :
*A représente un atome d'azote et E représente un groupement CH quand Y représente un groupement phényloxy éventuellement substitué,
*ou A représente un groupement CH et E représente un atome d'azote quand Y représente un groupement benzofuran-3-yl,
leurs hydrates, leurs solvates et leurs sels d'addition à un acide pharmaceutiquement acceptable.

5. Composés de formule (IB) selon la revendication 3 **caractérisés en ce que** :
*V représente une liaison simple quand Y représente un groupement benzofuran-3-yl,
*ou V représente une chaîne alkylène (C₁-C₄) linéaire ou ramifiée quand Y représente un groupement phényloxy éventuellement substitué,
leurs hydrates, leurs solvates et leurs sels d'addition à un acide pharmaceutiquement acceptable.

6. Composés de formule (I) selon la revendication 1 qui sont le :
- N-{2-[4-(phénoxyméthyl)-1-pipéridinyl]éthyl}-1-indolinecarboxamide,
- 5-fluoro-N-(2-{4-[2-(4-fluorophénoxy)éthyl]-1-pipéridinyl}éthyl)-1-indolecarboxamide,
- N-{2-[4-(*p*-fluorophénoxyméthyl)-1-pipéridinyl]éthyl}-1-indolinecarboxamide,
- N-{2-[4-(phénoxyméthyl)-1-pipéridinyl]éthyl}-1-indolecarboxamide,
leurs hydrates, leurs solvates et leurs sels d'addition à un acide pharmaceutiquement acceptable.

7. Procédé de préparation des composés de formule (I) selon la revendication 1 **caractérisé en ce qu**'on utilise comme produit de départ un composé de formule (XV) : 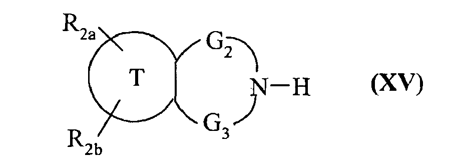 dans laquelle R₂ₐ, R_{2b}, G₂, G₃ et T ont les mêmes significations que dans la formule (I),
composé de formule (XV) que l'on traite soit par du diphosgène ou du triphosgène, en présence d'une base, soit par du di(1*H*-imidazol-1-yl)méthanone, pour conduire aux composés de formule (XVI) : 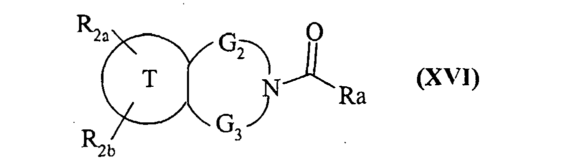 dans laquelle R₂ₐ, R_{2b}, G₂, G₃ et T sont tels que définis précédemment et Rₐ représente un atome de chlore, un groupement 1*H*-imidazol-1-yl ou
un groupement de formule : 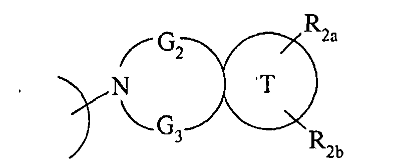 dans laquelle R₂ₐ, R_{2b}, G₂, G₃ et T sont tels que définis précédemment,
composé de formule (XVI) qui est mis à réagir avec un composé de formule (XVII) : 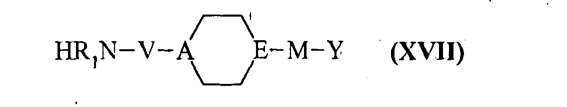 dans laquelle R₁, V, A, E, M et Y sont tels que définis dans la formule (I),
pour conduire aux composés de formule (I), que l'on purifie, le cas échéant, selon des techniques classiques de purification, dont on sépare éventuellement les isomères selon une technique classique de séparation, et que l'on transforme, si on le souhaite, en ses hydrates, ses solvates ou ses sels d'addition à un acide pharmaceutiquement acceptable.

8. Compositions pharmaceutiques contenant comme principe actif au moins un composé de formule (I) selon l'une quelconque des revendications 1 à 6, seul ou en combinaison avec un ou plusieurs excipients ou véhicules inertes, non toxiques, pharmaceutiquement acceptables.

9. Compositions pharmaceutiques selon la revendication 8 contenant au moins un principe actif selon l'une quelconque des revendications 1 à 6, utiles en tant que médicament pour le traitement des maladies ou des conditions pathologiques dans lesquelles une dysfonction endothéliale est connue.

10. Compositions pharmaceutiques selon la revendication 8 contenant au moins un principe actif selon l'une quelconque des revendications 1 à 6, utiles en tant que médicament pour prévenir le développement, l'extension et les complications des lésions athéroscléreuses, en particulier chez les patients présentant un facteur de risque vasculaire (dyslipidémie, diabète, hypertension artérielle systémique), pour prévenir les complications vasculaires après pontage vasculaire, dilatation vasculaire, reperméabilisation vasculaire et transplantation cardiaque, ou pour traiter l'ischémie myocardique ou périphérique, l'insuffisance cardiaque et l'hypertension artérielle pulmonaire.

## Claims

1. Compounds of formula (I) : 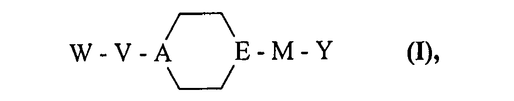 wherein :
V represents a single bond or a linear or branched (C₁-C₆)alkylene chain,
M represents a single bond or a linear or branched (C₁-C₄)alkylene chain,
A and E each represents a nitrogen atom or a CH group, but at least one of the two groups A or E represents a nitrogen atom,
W represents a group of formula (ii) : 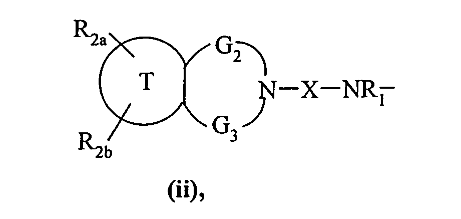
wherein:
X represents a carbonyl group,
G₂ represents a single bond or a methylene group,
G₃ represents:
*a linear (C₂-C₃)alkylene chain when G₂ represents a bond,
*or a linear (C₁-C₂)alkylene chain when G₂ represents a methylene group,
said alkylene chain in each of those cases optionally containing a double bond,
T represents a phenyl group fused with the ring to which it is attached,
R₁ represents a hydrogen atom or a methyl group,
R₂ₐ and R_{2b}, which are the same or different, each independently of the other represents a group selected from a hydrogen atom, a halogen atom, a linear or branched (C₁-C₆)alkyl group, a hydroxy group, a linear or branched (C₁-C₆)alkoxy group, a mercapto group, a linear or branched (C₁-C₆)alkylthio group, a linear or branched (C₁-C₆)trihaloalkyl group, a cyano group, a nitro group, an amino group, a linear or branched (C₁-C₆)alkylamino group, a di-(C₁-C₆)alkylamino group in which each alkyl moiety may be linear or branched, a linear or branched (C₁-C₆)trihaloalkoxy group, an aryloxy group, an aryl-(C₁-C₆)alkoxy group in which the alkoxy moiety is linear or branched, a linear or branched (C₁-C₆)alkylsulphonate group, a linear or branched (C₁-C₆)trihaloalkylsulphonate group and a linear or branched (C₁-C₆)-alkylsulphonyl group, or R₂ₐ + R_{2b}, taken together in adjacent positions, represent a group selected from methylenedioxy, 1,2-ethylenedioxy, 1,3-propylenedioxy, and ethylene optionally substituted by a group selected from cyano, hydroxymethyl, linear or branched (C₁-C₆)alkoxycarbonyl, aryloxycarbonyl and aryl-(C₁-C₆)alkoxycarbonyl in which the alkoxy moiety is linear or branched,
Y represents an aryloxy, heteroaryloxy or heteroaryl-B group, each of which groups may optionally be substituted by one or more groups, which may be the same or different, selected from among the definitions of R₂ₐ,
their hydrates, their solvates and addition salts thereof with a pharmaceutically acceptable acid,
it being understood that:
- an aryl group means a group selected from phenyl, biphenyl, naphthyl, dihydronaphthyl, tetrahydronaphthyl, indanyl, indenyl and benzocyclobutyl,
- a heteroaryloxy group means a monocyclic aromatic or bicyclic, 5- to 12-membered system attached to an oxygen atom, containing one or two hetero atoms, which may be the same or different, selected from oxygen, nitrogen and sulphur, and wherein, in the case of a bicyclic system, one of the rings has an aromatic character and the other ring may be aromatic or partially hydrogenated,
- a heteroaryl-B group means a monocyclic aromatic or bicyclic aromatic, 5- to 12-membered system containing from 1 to 3 hetero atoms, which may be the same or different, selected from oxygen, nitrogen and sulphur,
- an aryloxy group means an aryl group as defined hereinbefore attached to an oxygen atom, with the proviso that the compounds of formula (I) are other than 1-[2-[1-(1,4-benzodioxan-2-yl)methyl-4-piperidyl]ethylaminocarbonyl]-1,2,3,4-tetrahydroquinoline.

2. Compounds of formula (I) according to claim 1, **characterised in that** Y represents a benzofuran-3-yl group or a phenyloxy group optionally substituted by a group R₂ₐ as defined for formula (I), their hydrates, their solvates and addition salts thereof with a pharmaceutically acceptable acid.

3. Compounds of formula (I) according to claim 2, **characterised in that** they represent compounds of formula (IB) : 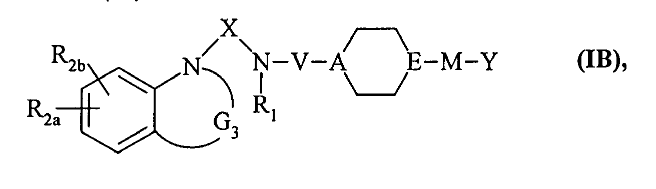 wherein :
R₂ₐ and R_{2b} are as defined for formula (I),
G₃ represents a group of formula -(CH₂)₂-, -(CH₂)₃- or -CH=CH-,
X represents a carbonyl group,
R₁ represents a hydrogen atom,
A, E and V are as defined for formula (I),
M represents a linear or branched (C₁-C₄)alkylene chain,
Y represents a benzofuran-3-yl group or a phenyloxy group optionally substituted by a group selected from a halogen atom, a linear or branched (C₁-C₆)alkylsulphonyl group and a linear or branched (C₁-C₆)alkylthio group.
their hydrates, their solvates and addition salts thereof with a pharmaceutically acceptable acid.

4. Compounds of formula (IB) according to claim 3, **characterised in that** :
* A represents a nitrogen atom and E represents a CH group when Y represents an optionally substituted phenyloxy group,
* or A represents a CH group and E represents a nitrogen atom when Y represents a benzofuran-3-yl group.
their hydrates, their solvates and addition salts thereof with a pharmaceutically acceptable acid.

5. Compounds of formula (IB) according to claim 3, **characterised in that**:
* V represents a single bond when Y represents a benzofuran-3-yl group,
* or V represents a linear or branched (C₁-C₄)alkylene chain when Y represents an optionally substituted phenyloxy group,
their hydrates, their solvates and addition salts thereof with a pharmaceutically acceptable acid.

6. Compounds of formula (I) according to claim 1 which are :
- N-{2-[4-(phenoxymethyl)-1-piperidyl]ethyl}-1-indolinecarboxamide,
- 5-fluoro-N-(2- {4-[2-(4-fluorophenoxy)ethyl]-1-piperidyl}ethyl)-1-indolecarboxamide,
- N- {2-[4-(*p*-fluorophenoxymethyl)-1-piperidyl]ethyl}-1-indolinecarboxamide,
- N- {2-[4-(phenoxymethyl)-1-piperidyl]ethyl}-1-indolecarboxamide,
their hydrates, their solvates and addition salts thereof with a pharmaceutically acceptable acid.

7. Process for the preparation of compounds of formula (I) according to claim 1, **characterised in that** there is used as starting material a compound of formula (XV): 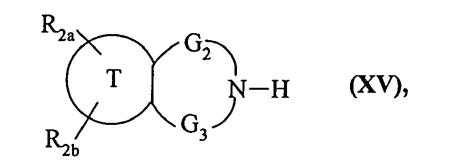 wherein R₂ₐ, R_{2b}, G₂, G₃ and T have the same meanings as in formula (I),
which compound of formula (XV) is treated either with diphosgene or triphosgene, in the presence of a base, or with di-(1*H*-imidazol-1-yl)methanone to yield the compounds of formula (XVI) : 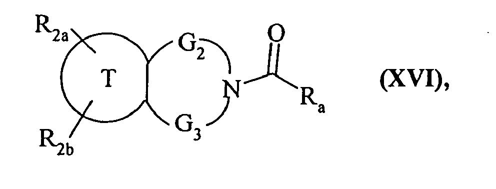 wherein R₂ₐ, R_{2b}, G₂, G₃ and T are as defined hereinbefore and Rₐ represents a chlorine atom, a 1*H*-imidazol-1-yl group or
a group of formula : 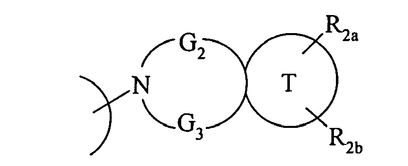 wherein R₂ₐ, R_{2b}, G₂, G₃ and T are as defined hereinbefore,
which compound of formula (XVI) is reacted with a compound of formula (XVII) : 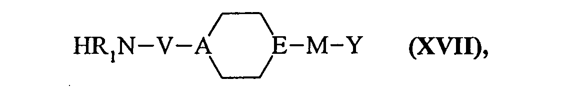 wherein R₁, V, A, E, M and Y are as defined for formula (I), to yield the compounds of formula (I), which are purified, if necessary, according to conventional purification techniques, are separated, where appropriate, into their isomers according to a conventional separation technique and are converted, if desired, into their hydrates, solvates or addition salts with a pharmaceutically acceptable acid.

8. Pharmaceutical compositions comprising as active ingredient at least one compound of formula (I) according to any one of claims 1 to 6, alone or in combination with one or more inert, non-toxic pharmaceutically acceptable excipients or carriers.

9. Pharmaceutical compositions according to claim 8 comprising at least one active ingredient according to any one of claims 1 to 6 for use as a medicament for the treatment of diseases or pathological conditions in which endothelial dysfunction is known.

10. Pharmaceutical compositions according to claim 8 comprising at least one active ingredient according to any one of claims 1 to 6 for use as a medicament for preventing the development, extension and complications of atherosclerotic lesions, especially in patients having a vascular risk factor (dyslipidaemia, diabetes, systemic arterial hypertension), for preventing vascular complications after vascular bypass, vascular dilatation, vascular repermeabilisation and heart transplantation or for treating myocardial or peripheral ischaemia, cardiac insufficiency and pulmonary arterial hypertension.

## Patentansprüche

1. Verbindungen der Formel (I): 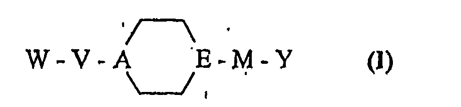 in der:
V eine Einfachbindung oder eine geradkettige oder verzweigte (C₁-C₆)-Alkylenkette bedeutet,
M eine geradkettige oder verzweigte (C₁-C₄)-Alkylenkette bedeutet,
A und E jeweils ein Stickstoffatom oder eine Gruppe CH bedeuten, wobei mindestens eine der beiden Gruppen A oder E ein Stickstoffatom darstellt,
W eine Gruppe der Formel (ii) bedeutet: 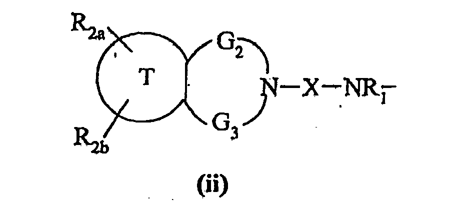 in der:
X eine Carbonylgruppe darstellt,
G₂ eine Einfachbindung oder eine Methylengruppe darstellt,
G₃ *eine geradkettige (C₂-C₃)-Alkylenkette darstellt, wenn G₂ eine Bindung bedeutet,
*oder eine geradkettige (C₁-C₂)-Alkylenkette darstellt, wenn G₂ eine Methylengruppe bedeutet,
wobei die Alkylenkette in jedem der Fälle gegebenenfalls eine Doppelbindung aufweist.
T eine Phenylgruppe darstellt, die mit dem Ring, an den sie gebunden ist, kondensiert ist,
R₁ ein Wasserstoffatom oder eine Methylgruppe darstellt,
R₂ₐ, R_{2b}, die gleichartig oder verschieden sind, unabhängig voneinander eine Gruppe bedeuten ausgewählt aus dem Wasserstoffatom, Halogenatomen, geradkettigen oder verzweigten (C₁-C₆)-Alkylgruppen, Hydroxygruppen, geradkettigen oder verzweigten (C₁-C₆)-Alkoxygruppen, Mercaptogruppen, geradkettigen oder verzweigten (C₁-C₆)-Alkylthiogruppen, geradkettigen oder verzweigten (C₁-C₆)-Trihalogenalkylgruppen, Cyanogruppen, Nitrogruppen, Aminogruppen, geradkettigen oder verzweigten (C₁-C₆)-Alkylaminogruppen, geradkettigen oder verzweigten Di-(C₁-C₆)-alkylaminogruppen, geradkettigen oder verzweigten (C₁₋C₆)-Trihalogenalkoxygruppen, Aryloxygruppen, geradkettigen oder verzweigten Aryl-(C₁-C₆)-alkoxygruppen, geradkettigen oder verzweigten (C₁-C₆)-Alkylsulfonatgruppen, geradkettigen oder verzweigten (C₁-C₆)-Trihalogenalkylsulfonatgruppen und geradkettigen oder verzweigten (C₁-C₆)-Alkylsulfonylgruppen, oder R₂ₐ + R_{2b}, wenn sie in benachbarten Positionen stehen, eine Gruppe darstellen ausgewählt aus Methylendioxy, 1,2-Ethylendioxy, 1,3-Propylendioxy und Ethylen, welches gegebenenfalls durch eine Gruppe ausgewählt aus Cyano, Hydroxymethyl, geradkettigem oder verweigtem (C₁-C₆)-Alkoxycarbonyl, Aryloxycarbonyl und geradkettigem oder verzweigtem Aryl-(C₁-C₆)-alkoxycarbonyl substituiert ist, und
Y eine Aryloxygruppe, Heteroaryloxygruppe oder Heteroaryl-B-gruppe bedeutet,
wobei jede dieser Gruppen gegebenenfalls durch eine oder mehrere, gleichartige oder verschiedenartige Gruppen ausgewählt aus den Definitionen von R₂ₐ substituiert ist,
deren Hydrate, deren Solvate sowie deren Additionssalze mit einer pharmazeutisch annehmbaren Säure,
wobei man unter einer:
- Arylgruppe eine Gruppe versteht, ausgewählt aus Phenyl, Biphenyl, Naphthyl, Dihydronaphthyl, Tetrahydronaphthyl, Indanyl, Indenyl und Benzocyclobutyl,
- Heteroaryloxygruppe ein monocyclisches aromatisches oder bicyclisches System versteht, das an ein Sauerstoffatom gebunden ist, welches System 5 bis 12 Kettenglieder aufweist und ein oder zwei gleichartige oder verschiedenartige Heteroatome ausgewählt aus Sauerstoff, Stickstoff und Schwefel enthält, und bei dem einer der Ringe im Fall eines bicyclischen Systems einen aromatischen Charakter besitzt, während der andere aromatisch oder teilweise hydriert sein kann,
- Heteroaryl-B-gruppe ein monocyclisches aromatisches oder bicyclisches aromatisches System mit 5 bis 12 Kettengliedern versteht, welches 1 bis 3 gleichartige oder verschiedenartige Heteroatome ausgewählt aus Sauerstoff, Stickstoff und Schwefel enthält,
- Aryloxygruppe eine Arylgruppe versteht, wie sie oben definiert worden ist, die an ein Sauerstoffatom gebunden ist,
mit der Maßgabe, daß die Verbindungen der Formel (I) von 1-[2-[1-(1,4-Benzodioxan-2-yl)-methyl-4-piperidinyl]-ethylaminocarbonyl]-1,2,3,4-tetrahydrochinolin verschieden sind.

2. Verbindungen der Formel (I) nach Anspruch 1, **dadurch gekennzeichnet, daß** Y eine Benzofuran-3-yl-gruppe oder eine Phenyloxygruppe, die gegebenenfalls durch eine Gruppe R₂ₐ, wie sie bezüglich der Formel (I) definiert worden ist, substituiert ist, bedeutet, deren Hydrate, deren Solvate und deren Additionssalze mit einer pharmazeutisch annehmbaren Säure.

3. Verbindungen der Formel (I) nach Anspruch 1, **dadurch gekennzeichnet, daß** sie der Formel (IB) entsprechen: 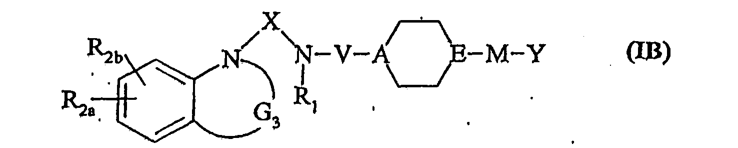 in der:
R₂ₐ und R_{2b} die bezüglich der Formel (I) angegebenen Bedeutungen besitzen,
G₃ eine Gruppe der Formeln -(CH₂)₂-, -(CH₂)₃- oder -CH=CH- darstellt,
X eine Carbonylgruppe bedeutet,
R₁ ein Wasserstoffatom darstellt,
A, E und V die bezüglich der Formel (I) angegebenen Bedeutungen besitzen,
M eine geradkettige oder verzweigte (C₁-C₄)-Alkylenkette darstellt,
Y eine Benzofuran-3-yl-gruppe oder eine Phenyloxygruppe, die gegebenenfalls durch eine Gruppe ausgewählt aus Halogenatomen, geradkettigen oder verzweigten (C₁-C₆)-Alkylsulfonylgruppen und geradkettigen oder verzweigten (C₁-C₆)-Alkylthiogruppen substituiert ist, bedeutet,
deren Hydrate, deren Solvate und deren Additionssalze mit einer pharmazeutisch annehmbaren Säure.

4. Verbindungen der Formel (IB) nach Anspruch 3, **dadurch gekennzeichnet, daß**:
* A ein Stickstoffatom und E eine Gruppe CH bedeuten, wenn Y eine gegebenenfalls substituierte Phenyloxygruppe darstellt,
* oder A eine Gruppe CH und E ein Stickstoffatom bedeuten, wenn Y eine Benzofuran-3-yl-gruppe darstellt,
deren Hydrate, deren Solvate sowie deren Additionssalze mit einer pharmazeutisch annehmbaren Säure.

5. Verbindungen der Formel (IB) nach Anspruch 3, **dadurch gekennzeichnet, daß**:
* V eine Einfachbindung bedeutet, wenn Y eine Benzofuran-3-yl-gruppe darstellt,
* oder V eine geradkettige oder verzweigte (C₁-C₄)-Alkylenkette bedeutet, wenn Y eine gegebenenfalls substituierte Phenyloxygruppe darstellt,
deren Hydrate, deren Solvate und deren Additionssalze mit einer pharmazeutisch annehmbaren Säure.

6. Verbindungen der Formel (I) nach Anspruch 1, nämlich:
- N-{2-[4-(Phenoxymethyl)-1-piperidinyl]-ethyl}-1-indolincarboxamid,
- 5-Fluor-N-(2-{4-[2-(4-fluorphenoxy)-ethyl]-1-piperidinyl}-ethyl)-1-indolcarboxamid,
- N-{2-[4-(*p*-Fluorphenoxymethyl)-1-piperidinyl]-ethyl}-1-indolincarboxamid,
- N-{2-[4-(Phenoxymethyl)-1-piperidinyl]-ethyl}-1-indolcarboxamid,
deren Hydrate, deren Solvate und deren Additionssalze mit einer pharmazeutisch annehmbaren Säure.

7. Verfahren zur Herstellung der Verbindungen der Formel (I) nach Anspruch 1, **dadurch gekennzeichnet, daß** man als Ausgangsprodukt eine Verbindung der Formel (XV) verwendet: 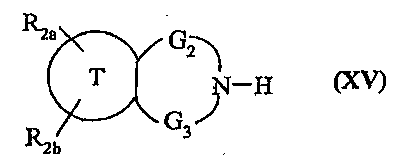 in der R₂ₐ, R_{2b}, G₂, G₃ und T die bezüglich der Formel (I) angegebenen Bedeutungen besitzen,
welche Verbindung der Formel (XV) man entweder mit Diphosgen oder Triphosgen in Gegenwart einer Base oder mit Di-(1*H*-imidazol-1-yl)-methanon behandelt zur Bildung der Verbindungen der Formel (XVI): 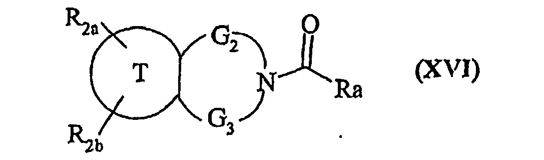 in der R₂ₐ, R_{2b}, G₂, G₃ und T die oben angegebenen Bedeutungen besitzen und Rₐ ein Chloratom, eine 1*H*-Imidazol-1-yl-gruppe oder
eine Gruppe der Formel: 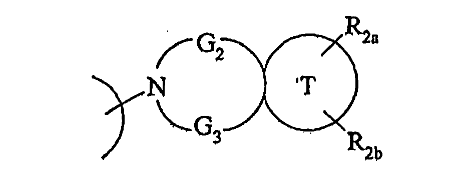 in der R₂ₐ, R_{2b}, G₂, G₃ und T die oben angegebenen Bedeutungen besitzen, darstellt,
welche Verbindung der Formel (XVI) mit einer Verbindung der Formel (XVII): 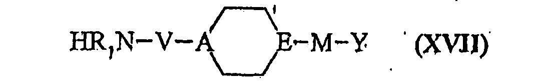 in der R₁, V, A, E, M und Y die bezüglich der Formel (I) angegebenen Bedeutungen besitzen, umgesetzt wird
zur Bildung der Verbindungen der Formel (I), welche man gegebenenfalls mit Hilfe klassischer Reinigungsmethoden reinigt, welche man gegebenenfalls mit Hilfe einer klassischen Trennmethode in ihre Isomeren auftrennt und welche man gewünschtenfalls in ihre Hydrate, Solvate oder Additionssalze mit einer pharmazeutisch annehmbaren Säure überführt.

8. Pharmazeutische Zubereitungen enthaltend als Wirkstoff mindestens eine Verbindung der Formel (I) nach einem der Ansprüche 1 bis 6, allein oder in Kombination mit einem oder mehreren inerten, nichttoxischen, pharmazeutisch annehmbaren Trägermaterialien oder Hilfsstoffen.

9. Pharmazeutische Zubereitungen nach Anspruch 8, enthaltend mindestens einen Wirkstoff nach einem der Ansprüche 1 bis 6, nützlich als Arzneimittel zur Behandlung von Krankheiten oder pathologischen Zuständen, für die eine endotheliale Dysfunktion bekannt ist.

10. Pharmazeutische Zubereitungen nach Anspruch 8, enthaltend mindestens einen Wirkstoff nach einem der Ansprüche 1 bis 6, nützlich als Arzneimittel zur Vorbeugung der Entwicklung, der Verschlimmerung und von Komplikationen von atherosklerösen Läsionen, insbesondere bei Patienten, die einen Gefäßrisikofaktor aufweisen (Dyslipidämie, Diabetes, arterielle systemische Hypertension), zur Vorbeugung von Gefäßkomplikationen nach Gefäßüberbrückungen, Gefäßerweiterungen, Gefäß-Repermeabilisierungen und Herztransplantationen oder zur Behandlung von myokardischer oder peripherer Ischämie, Herzinsuffizienz und arterieller pulmonarer Hypertension.
